# EUROPÄISCHE PATENTANMELDUNG

(11) **EP 3 808 267 A1**
(43) Veröffentlichungstag der Anmeldung: **21.04.2021**
(21) Anmeldenummer: 19204063.2
(22) Anmeldetag: 18.10.2019
(51) Int. Cl.: A61B 5/107

(54) **VERFAHREN ZUR BESTIMMUNG ANATOMISCHER SYMMETRIEELEMENTE**

(71) Anmelder: Probio-Dental Medical Developments UG (Haftungsbeschränkt), 67063 Ludwigshafen (DE)
(72) Erfinder: SCHELL, Ingo, 67063 Ludwigshafen (DE); RAUSCH, Claus-Uwe, 34131 Kassel (DE)
(74) Vertreter: Reitstötter Kinzebach

(57) **Zusammenfassung**

Die vorliegende Erfindung betrifft ein Verfahren zur Bestimmung der therapeutischen Lage und Ausrichtung wenigstens eines anatomischen Symmetrieelements eines Patienten, die Verwendung dieses Verfahrens für die Herstellung patientenindividueller Aufbissschienen, für die Planung und Berechnung von Zahnprothetik und für die patientenindividuellen Modifikation von Artikulatoren, eine Vorrichtung, die in diesem Verfahren einsetzbar ist, sowie einen Bausatz und ein Verfahren für die patientenindividuelle Modifikation eines konfektionierten Artikulators.

## Beschreibung

Die vorliegende Erfindung betrifft ein Verfahren zur Bestimmung der therapeutischen Lage und Ausrichtung wenigstens eines anatomischen Symmetrieelements eines Patienten, die Verwendung dieses Verfahrens für die Herstellung patientenindividueller Aufbissschienen, für die Planung und Berechnung von Zahnprothetik und für die patientenindividuellen Modifikation von Artikulatoren, eine Vorrichtung, die in diesem Verfahren einsetzbar ist, sowie einen Bausatz und ein Verfahren für die patientenindividuelle Modifikation eines konfektionierten Artikulators.

### Hintergrund der Erfindung

Der menschliche Körper besitzt eine bilaterale Symmetrie, d. h. er ist durch eine sagittale Ebene in eine rechte und linke Hälfte geteilt, die sich spiegelbildlich zueinander verhalten. Der Bewegungsapparat des Menschen ist daher bilateral symmetrisch aufgebaut. Des Weiteren ist die Position und Ausrichtung der einzelnen Strukturelemente des menschlichen Bewegungsapparates im Wesentlichen funktional bedingt und unterliegen biomechanischen Prinzipien. Die Gestalt des menschlichen Bewegungsapparates sowie die Position und Ausrichtung der darin befindlichen anatomischen Strukturelemente lassen sich daher anhand mathematischer Gesetzmäßigkeiten beschreiben. So werden in der Literatur zur Beschreibung des menschlichen Körpers anatomische Symmetrieelemente, wie anatomische Ebenen, Achsen und Kugeln verwendet. Umgekehrt lassen sich die physiologisch "ideale" Lagen und Ausrichtungen einzelner Strukturelemente des menschlichen Bewegungsapparates mithilfe solcher anatomischen Symmetrieelemente bestimmen.

Insbesondere bei der Planung und Durchführung wirksamer Therapien zur Behandlung von Erkrankungen, welche die Körperhaltung bzw. die Körperstatik, wie beispielsweise die Ausrichtung des Kopfes und der Wirbelsäule, insbesondere im Nackenbereich, betreffen, und bei der Planung von Prothesen oder Orthesen sind Kenntnis der physiologischen/therapeutischen Lage und Ausrichtung von anatomischen Symmetrieelementen essentiell, um wirksame Therapien bereitstellen zu können. Daneben ist die Kenntnis über die physiologische/therapeutischen Lage und Ausrichtung solcher anatomischen Symmetrieelemente auch im Bereich der Körperdynamik nützlich, zum Beispiel bei der Erkennung und Behebung fehlerhafter Bewegungen, die zu Fehlbelastungen oder Überbelastungen führen oder zur Verbesserung der Bewegungsabläufe bei sportlichen Tätigkeiten.

Eine häufig anzutreffende Ursache von Erkrankungen, welche die Körperhaltung bzw. die Körperstatik betreffen, sind funktionelle Störungen des Kauorgans, die von einer Fehlstellung des Kiefergelenkes ausgehen. Auch bei diesen Erkrankungen sind möglichst genaue Kenntnisse zur physiologische/therapeutischen Lage der hierfür relevanten anatomischen Strukturen, wie insbesondere die physiologische Lage des Ober- und Unterkiefers zueinander bzw. die physiologische Lage des Kiefergelenkes, essentiell. Die Lage dieser anatomischen Strukturen lässt sich wiederum aus den Positionen und Ausrichtungen anatomischer Symmetrieelemente, wie beispielsweise der Körpermittelachse (Mittelmeridian), die Kiefergelenkachse und die Okklusionsebene, ableiten. Ist die physiologische/therapeutische Lage und Ausrichtung dieser anatomischen Symmetrieelemente ermittelt, kann basierend darauf auch die physiologische Position und Ausrichtung der Kiefer und des Kiefergelenks des Patienten berechnet, und entsprechende therapeutische Maßnahmen ergriffen werden, wie beispielsweise die Erstellung von Aufbissschienen und/oder Zahnersatz, usw. In der Regel sind die getroffenen Maßnahmen umso wirksamer je exakter die therapeutische Lage dieser anatomischen Symmetrieelemente bestimmt wurde.

Im Stand der Technik sind Verfahren zur Bestimmung der Lage anatomischer Symmetrieelemente im Kopf- und Halsbereich bekannt.

F. Hornung et al., J. Compr. Dentof. Orthod. + Orthop. (COO) Umf. Dentof. Orthod. u. Kieferorthop. (UOO), 2016 (c), No. 3-4, S. 30-48, beispielsweise beschreiben ein Verfahren zur Bestimmung zahnmedizinisch relevanter cranialer Symmetrieelemente basierend auf der Symmetrie zwischen den Sinnesorganen des menschlichen Schädels (CranioPlan®-Verfahren). Bei diesem Verfahren wird ausgehend von der dreidimensionalen Knochenstruktur des Schädels des Patienten zunächst eine Trapezfläche (die sogenannte Cranial-Ebene oder "Cranial Plane" (CP)) bestimmt. Diese wird aus dem Maximum der Hornhautkrümmungen des rechten und linken Auges und den Zentren des Amboss des linken und des rechten Ohres gebildet. Hieraus werden anschließend der geometrische Schwerpunkt der Trapez-Fläche, i.e. der Craniale Achsenpunkt ("Cranial Axis Point" (CAP)), der Kreuzungspunkt der Trapez-Diagonalen ("Cranial Cross Point" (CCP)), die senkrechte Achse auf der CP durch den CAP ("Cranial Symmetry Axis" (CSA)) sowie die senkrechte Fläche auf der CP durch die Streckenmittelpunkte des Augenabstandes und des Amboss-Abstandes ("Cranial Symmetry Plane" (CSP)) bestimmt. Aus diesen cranialen Symmetrieelementen lassen sich zahnmedizinisch relevante Symmetrieelemente des Schädels, wie beispielsweise die Atlaskugel, die Kiefergelenksachse und die Okklusionsebene, ableiten.

Bei den im Stand der Technik bekannten Verfahren werden in aller Regel dreidimensionale Bildgebungsverfahren eingesetzt, wie z. B. dreidimensionales Röntgen, Kernspintomographie oder Magnetresonanztomographie (MRT), dreidimensionaler Computertomographie (CT) bzw. digitale Volumentomographie (DVT). Gegebenenfalls werden zusätzlich die Bewegungsbahnen des Unterkiefers mittels klinischer Okklusionsanalyse und/oder digitaler achsiographischer Untersuchungsverfahren ermittelt. Auf der Grundlage dieser Daten wird dann eine vermutete therapeutische/physiologische Lage der Kiefer und der Kiefergelenke ermittelt, wie zuvor beispielhaft anhand des CranioPlan®-Verfahren dargelegt. Hierzu wird die vermutete therapeutische/physiologische Lage der Kiefer und der Kiefergelenke entweder aus den anatomischen Gegebenheiten des Patienten, wie z. B. aus verschiedenen anatomischen Fixpunkten in der Knochenstruktur des Patienten, und unter Zuhilfenahme von Normwerten für bestimmte anatomische Abstände und Winkel berechnet oder es werden digitaler Modelle von dem behandelnden Kieferorthopäden von Hand in die dreidimensionale Knochenstruktur des Patienten eingepasst.

Die im Stand der Technik bekannten Verfahren zur Bestimmung der physiologischen/therapeutischen Lage und Ausrichtung der Kiefer und der Kiefergelenke weisen mehrere Nachteile auf.

Zunächst einmal ist die Genauigkeit der bekannten Verfahren begrenzt, da die Bestimmung der therapeutischen Lage und Ausrichtung der relevanten anatomischen Symmetrieelemente zur Ermittlung der therapeutische/physiologische Lage der Kiefer und der Kiefergelenke anhand der Knochenstruktur des Patienten erfolgt. Die Knochenstruktur eines Menschen besitzt jedoch von Natur aus keine perfekte Symmetrie. Dies trifft auch auf die Symmetrie zwischen den Sinnesorganen des menschlichen Schädels zu. Zudem kann die Knochenstruktur durch Krankheit und/oder Unfälle verändert sein. Des Weiteren kann die Erfassung der Knochenstruktur mittels röntgenbasierter bildgebender Verfahren durch die Anwesenheit von Metallteilen und/oder Keramikteilen erheblich erschwert sein (Erzeugung von Artefakten). D. h. die Position der einzelnen anatomischen Symmetrieelemente lässt sich nicht in dem Maße exakt und zuverlässig bestimmen, wie dies für eine möglichst effiziente patientenindividuelle Therapie wünschenswert wäre. Das Weitere berücksichtigen die bekannten Verfahren die Gesamtkörperhaltung des Patienten nur unzureichend, da der Fokus dieser Verfahren üblicherweise auf den Kopf und Nackenbereich des Patienten liegt. Zudem sind diese Verfahren in der Regel teuer und zeitaufwändig, da zur Bestimmung der Position und Ausrichtung der Kiefer und Nackenwirbel aufwändige dreidimensionale Bildgebungsverfahren eingesetzt werden. Da zur dreidimensionalen Bestimmung der Knochenstruktur oft Röntgenstrahlen eingesetzt werden, ist auch die Strahlenbelastung für den Patienten in der Regel recht hoch.

Es ist daher die Aufgabe der vorliegenden Erfindung, ein Verfahren zur Bestimmung der therapeutischen Lage und Ausrichtung wenigstens eines anatomischen Symmetrieelements eines Patienten, insbesondere zur Herstellung patientenindividueller Aufbissschienen, zur Planung und Berechnung von Zahnprothetik und zur patientenindividuelle Modifikation von Artikulatoren, bereitzustellen, welches gegenüber den im Stand der Technik bekannten Verfahren eine schnellere, präzisere und individuellere Behandlung des Patienten ermöglicht. Daneben soll das Verfahren ohne den Einsatz teurer Diagnosevorrichtungen durchführbar sein.

Diese Aufgabe wird erfindungsgemäß durch ein Verfahren zur Bestimmung der therapeutischen Lage und Ausrichtung wenigstens eines anatomischen Symmetrieelements eines Patienten, gelöst, wie in den Patentansprüchen und im Folgenden detailliert beschrieben wird.

Das erfindungsgemäße Verfahren zeichnet sich insbesondere dadurch aus, dass die therapeutische Lage und Ausrichtung der relevanten anatomischen Symmetrieelemente eines Patienten schnell, zuverlässig und mit hoher Präzision bestimmt werden können. Das erfindungsgemäße Verfahren kommt zudem ohne teure dreidimensionale Bildgebungsverfahren aus. In dem Verfahren werden insbesondere keine Röntgenstrahlen eingesetzt, wodurch sich dieses auch für den Einsatz bei Kindern und Jugendlichen eignet. Insbesondere ermöglicht das erfindungsgemäße Verfahren Vorsorgeuntersuchungen bei Kindern und Jugendlichen noch bevor mit kieferorthopädischen Behandlungen begonnen werden kann, d. h. üblicherweise vor der zweiten Phase des Zahnwechsels, wodurch Fehlbelastungen oder Überbelastungen früh erkannt werden können. Das erfindungsgemäße Verfahren beschränkt sich nicht nur auf die Kopfhaltung, sondern schließt die gesamte Körperhaltung bzw. Körperstatik des Patienten mit ein. Das erfindungsgemäße Verfahren bietet somit die Möglichkeit einer kieferorthopädischen Therapie, bei der die gesamte Positur des Patienten berücksichtigt wird (Funktionskieferorthopädie). Die für die Durchführung des erfindungsgemäßen Verfahrens benötigten Diagnosevorrichtungen sind vergleichsweise billig. Darüber hinaus können bei dem erfindungsgemäßen Verfahren alle Verfahrensschritte - von der Erhebung Patientenspezifischer Daten bis hin zur Erstellung von STL-Datensätzen für Aufbissschienen, Prothetik und/oder Arktikulatoren - in einem einzigen zusammenhängenden digitalen Arbeitsprozess durchgeführt werden. Hierbei können auch Daten bzw. Datensätze aus anderen Diagnoseverfahren mit einbezogen werden, selbst wenn diese auf unterschiedlichen Koordinatenbezugssystemen basieren.

### Zusammenfassung der Erfindung

Die vorliegende Erfindung basiert auf einer von den Erfindern entwickelten mathematischen Matrix, mit deren Hilfe aus wenigen anatomischen Fixpunkten, die sich aus einem biometrischen Fotos der Frontansicht des Kopfes des Patienten herauslesen lassen, die physiologisch bzw. therapeutische ("korrekte") Lage und Ausrichtung anatomischer Symmetrieelemente des Patienten mit hoher Präzision berechnen lassen; und zwar ohne das hierzu dreidimensionale Strukturen des Bewegungsapparates des Patienten, wie beispielsweise die dreidimensionale Knochenstruktur des Schädels des Patienten, erfasst werden müssen.

Die vorliegende Erfindung betrifft daher ein Verfahren zur Bestimmung der therapeutischen Lage und Ausrichtung wenigstens eines anatomischen Symmetrieelements eines Patienten, umfassend die folgenden Schritte:
a) Sicherstellung einer ausbalancierten Körperstatik des Patienten in aufrechter gerader Position,
b) Erstellen eines biometrischen Fotos der Frontansicht des Kopfes des Patienten,
   und
c) computergestützte Berechnung der therapeutischen Lage und Ausrichtung wenigstens eines anatomischen Symmetrieelements des Patienten anhand des in Schritt b) erstellten biometrischen Fotos unter Zuhilfenahme einer mathematischen Matrix.

Das vorliegende Verfahren lässt sich bevorzugt für die Herstellung patientenindividueller Aufbissschienen, für die Planung und Berechnung von Zahnprothetik und für die patientenindividuelle Modifikation von Artikulatoren einsetzten, insbesondere für Herstellung von Aufbissschienen zur Verwendung in der Behandlung eines Patienten mit wenigstens einer Erkrankung, die von einer funktionellen Störung des Kauorgans ausgeht.

Eine Ausführungsform der vorliegenden Erfindung betrifft somit ein Verfahren zur Herstellung von Aufbissschienen zur Verwendung in der Behandlung eines Patienten mit wenigstens einer Erkrankung, die von einer funktionellen Störung des Kauorgans ausgeht, umfassend die folgenden Schritte:
i) computergestützte Bestimmung der therapeutischen Lage und Ausrichtung wenigstens eines für die Stellung der Kiefer relevanten anatomischen Symmetrieelements des Patienten wie zuvor und im Folgenden definiert,
ii) computergestützte lagerichtige Zuordnung digitalisierter Ober- und Unterkiefermodelle zu den in Schritt i) bestimmten Symmetrieelementen, und
iii) Erzeugung wenigstens eines STL-Datensatzes der Aufbissschiene.

Des Weiteren betrifft die Erfindung eine Vorrichtung zur Sicherstellung einer ausbalancierten Körperstatik des Patienten in aufrechter gerader Position und zur Erstellung eines biometrischen Fotos der Vorderansicht des Kopfes eines Patienten, umfassend:
- eine erste digitale Kamera zur Aufnahme eines biometrischen Fotos der Frontansicht des Kopfes des Patienten in aufrechter gerader Position,
- ein Mittel zum Ausgleich der Längendifferenz der Beine des Patienten,
- wenigstens ein Mittel zum Auffinden einer ausbalancierten Körperstatik des Patienten, und
- wenigstens ein Mittel zur waagerechten Ausrichtung der Vorrichtung.

Des Weiteren betrifft die Erfindung einen Bausatz für die patientenindividuelle Modifikation eines konfektionierten Artikulators, umfassend:
- wenigstens ein Bauteil zur Fixierung des therapeutischen Abstandes der Kiefermodelle zur Scharnierachse des Artikulators,
- wenigstens ein Bauteil zur Fixierung der therapeutischen Ausrichtung der Kiefermodelle zur Scharnierachse des Artikulators.

Des Weiteren betrifft die Erfindung ein Verfahren zur patientenindividuellen Modifikation eines konfektionierten Artikulators umfassend die folgenden Schritte:
1) Berechnung eines patientenindividuellen Bauteils oder mehrerer patientenindividueller Bauteile (Bausatz) zur Modifikation eines konfektionierten Artikulators, unter Verwendung des zuvor und im Folgenden definierten Verfahrens,
2) Erzeugung der in Schritt 1) berechneten patientenindividuellen Bauteile,
3) Einbau der in Schritt 2) erzeugten patientenindividuellen Bauteile in den konfektionierten Artikulator.

### Detaillierte Beschreibung der Erfindung

Der Begriff "Okklusionsebene", nachfolgend auch als "Kauebene" bezeichnet, beschreibt die räumliche Ebene, auf der sich die Zähne des Ober- und Unterkiefers treffen.

Die Begriffe "therapeutisch" und "physiologisch" die unter anderem im Zusammenhang mit der Lage, Position, Stellung und/oder Ausrichtung anatomischer Symmetrieelemente und anatomischer Strukturelemente, wie die Kiefer, Kiefergelenke oder Halswirbel, verwendet werden, bezeichnen die jeweils für den Patienten aus anatomischer und biomechanischer Sicht ideale Lage, Position, Stellung und/oder Ausrichtung dieser anatomischen Symmetrie- und Strukturelemente.

Der Begriff "dreidimensionales Bildgebungsverfahren" bezeichnet jegliche bildliche Wiedergabe eines realen Objektes in drei bis vier Dimensionen, z. B. durch x-, y-, z- und Zeit-Koordinaten. Dreidimensionale Bildgebungsverfahren umfassen beispielsweise dreidimensionales Röntgen, Kernspintomographie oder Magnetresonanztomographie (MRT), dreidimensionaler Computertomographie (CT) bzw. digitale Volumentomographie und/oder Oberflächenrasterung durch Auflichtscannen.

Der Begriff "STL-Datensatz" bezeichnet Datensätze, die Informationen zum 3D Volumen von Bauteilen, die auf 3D Druckern im additiven Fertigungsverfahren produziert werden, enthalten. Das STL-Format ist das Standartformat zur Übertragung von Daten für solche additiven Fertigungsverfahren. STL-Datensätze werden aus 3D-CAD-Daten der jeweils zu druckenden Bauteile mittels Triangulation erzeugt, d. h. die kontinuierliche innere als auch äußere Freiformoberfläche der Bauteile wird rechnerisch durch Dreiecke angenähert.

Die Berechnungen der therapeutische bzw. physiologische Lage und/oder Ausrichtung der anatomischen Symmetrie- und Strukturelemente mit Hilfe einer mathematischen Matrix sind sehr komplex und rechenintensiv. Zudem sollen die einzelnen Verfahrensschritte möglichst schnell und automatisiert in einem einzigen Arbeitsfluss durchführbar sein. Daher werden die einzelnen Schritte des erfindungsgemäßen Verfahrens computergestützt durchgeführt, wobei die mathematische Matrix in einem Computerprogramm eingebettet ist, welches die einzelnen Programmschritte steuert.

### Schritt a):

Für die korrekte Bestimmung der physiologischen Lage und Ausrichter der anatomischen Symmetrieelemente des Patienten ist es essentiell, dass der Patient eine aufrechte gerade Körperhaltung einnimmt in der sich die Körperstatik in einem ausbalancierten Zustand befindet.

In einem ersten Schritt des erfindungsgemäßen Verfahrens wird der Patient daher in eine aufrechte gerade Haltung gebracht und so positioniert, dass eine möglichst ausbalancierte Körperstatik erreicht wird.

Hierzu müssen etwaige Haltungsstörungen, wie beispielsweise Fehlstellungen des Beckens, z. B. verursacht durch unterschiedliche Beinlängen, oder Fehlhaltungen des Kopfes, erkannt und möglichst ausgeglichen werden, da diese einen wesentlichen Einfluss auf die Körperstatik und damit indirekt auch auf die Stellung der Kiefer und des Kiefergelenks haben.

Zusätzlich sollten sich die Kiefergelenke und die Kaumuskulatur in einer entspannten Position bzw. in einem entspannten Zustand befinden, idealerweise in der sogenannten Ruheschwebelage. Dies kann mit speziellen Lockerungsmaßnahmen erreicht werden, wie beispielsweise vom Patienten durchgeführte Lockerungsübungen, die Durchführung einer Elektromyographie (EMG) im Kopfbereich des Patienten, oder durch Verabreichen einer Kaumasse, die der Patient wie ein herkömmliches Kaugummi kauen kann, bzw. Kombinationen der genannten Maßnahmen. Als besonders einfache und zweckmäßige Maßnahme zum Auffinden der Ruheschwebelage hat sich das Kauen einer Kaumasse durch den Patienten erwiesen, da sich hierdurch etwaige vorhandene Spannungen in der Kaumuskulatur schnell und effizient lösen. Nach dem Kauen nimmt der Patient eine entspannte leicht geöffnete Bissposition ein, wobei die Kaumasse zwischen den Zähnen verbleibt und die entspannte Position fixiert. Diese Position entspricht üblicherweise recht genau der patientenindividuellen Ruheschwebelage der Kiefer.

Gegebenenfalls werden Lippenspreizer zur Sichtbarmachung der Zähne bzw. der Zahnreihen eingesetzt. Dies hat den Vorteil, dass sich die nachfolgend berechneten therapeutischen Lagen der anatomischen Symmetrieelemente des Patienten, wie z. B. die Gaumenkugel, craniale Symmetrieachse bzw. Körpermittelachse (Mittelmeridian), die Kiefergelenksachse oder die Okklusionsebene, die auf dem digitalisierten biometrischen Foto der Frontansicht des Kopfes des Patienten dargestellt werden können, direkt mit der momentan vorliegenden Lage der Kiefer und Zahnreihen des Patienten vergleichen lassen. Dies ermöglicht eine erste visuelle Einschätzung der Diskrepanzen zwischen der momentanen Lage und der (gewünschten) therapeutischen Lage dieser anatomischen Strukturelemente der Patienten. Zusätzlich kann wenigstens ein im Lippenspreizer enthaltener und/oder am Lippenspreizer angebrachter Formkörper mit bekannter Größe, als Referenzmaß für die Skalierung des biometriechen Fotos eingesetzt werden.

Bevorzugt umfasst die Sicherstellung einer ausbalancierten Körperstatik des Patienten in Schritt a) wenigstens eine der folgenden Maßnahmen:
a.1) Einsatz einer Standunterlage zum Ausgleich der Längendifferenz der Beine des Patienten,
a.2) Ausrichtung der Kopf- und/oder Körperhaltung unter Zuhilfenahme eines Lots, und
a.3) gegebenenfalls die waagerechte Ausrichtung des Unterkiefers.

Patienten mit unterschiedlichen Beinlängen sind in der Praxis häufig anzutreffen. In dieser bevorzugten Ausführungsform des erfindungsgemäßen Verfahrens wird daher zum Ausgleich der Längendifferenz der Beine eine Standunterlage eingesetzt.

Als Standunterlage, nachfolgend auch als "Balance-Pad" bezeichnet, eignen sich in der Regel alle Unterlagen mit denen sich die Längendifferenz der Beine ausgleichen lassen. Bevorzugt handelt es sich hierbei um ein Material, das unter Druck nachgibt, wodurch die Füße bei einer etwaig vorhandenen Beinlängendifferenz unterschiedlich tief einsinken bis eine gleichmäßige Druckverteilung auf beiden Füßen vorliegt. Viskoelastische Materialien eignen sich aufgrund ihres viskosen Materialverhaltens unter Druck besonders gut für diesen Zweck. Mit diesen Materialien lassen sich Beinlängenunterschiede in idealer und einfacher Weise ausgleichen. Bei der erfindungsgemäß eingesetzten "Balance-Pad" handelt es sich daher insbesondere um ein viskoelastisches Material.

Damit eine aufrechte gerade Körperhaltung, bei der sich die Körperstatik in einem ausbalancierten Zustand befindet, effizient aufgefunden werden kann, wird in dieser bevorzugten Ausführungsform des erfindungsgemäßen Verfahrens wenigstens ein Lot vor den Patienten positioniert. Mit dessen Hilfe kann die Kopf- und Körperhaltung des Patienten gezielt ausgerichtet werden. Besonders bevorzugten wird das Lot vor einem Einwegspiegel positioniert, in dem sich der Patient betrachten kann. Diese Orientierungshilfe ermöglicht es dem Patienten seine Körperhaltung selbstständig auszurichten und eine aufrechte gerade Körperhaltung, bei der sich die Körperstatik in einem ausbalancierten Zustand befindet, selbstständig aufzufinden.

Falls erforderlich wird in dieser bevorzugten Ausführungsform der Unterkiefer des Patienten, möglichst waagerecht ausgerichtet. Hierzu wird üblicherweise ein Stativ, insbesondere ein höhenverstellbares Stativ, mit einer daran befestigten Kinnstütze als Orientierungshilfe für den Patienten eingesetzt. Hierbei soll der Patient sein Kinn nicht auf die Kinnstütze auflegen, sondern mit dem Kinn die Kinnstütze lediglich berühren, da dies ansonsten die Körperhaltung des Patienten beeinflussen würde.

In einer besonders bevorzugten Ausführungsform des erfindungsgemäßen Verfahrens umfasst die Sicherstellung einer ausbalancierten Körperstatik alle zuvor beschriebenen Maßnahmen a.1), a.2) und a.3).

Des Weiteren ist es vorteilhaft zur Durchführung des Verfahrens, wenn der Untergrund, auf dem der Patient steht, möglichst waagerecht ausgerichtet ist.

In einer speziellen Ausführungsform des erfindungsgemäßen Verfahrens umfasst die Sicherstellung einer ausbalancierten Körperstatik daher zusätzlich zu den Maßnahmen a.1), a.2) und a.3) die Sicherstellung der waagerechten Ausrichtung des Untergrundes, auf dem der Patient steht.

Zur waagerechten Ausrichtung des Untergrundes, kann beispielsweise eine auf den Boden aufgelegte, waagerecht ausrichtbare Bodenplatte eingesetzt werden, auf die sich der Patient während der Durchführung des Verfahrens stellen kann. Als waagerecht ausrichtbare Bodenplatte kann beispielsweise eine Schwerlastwaage eingesetzt werden.

In einer speziellen Ausführungsform des erfindungsgemäßen Verfahrens werden in Schritt a) zusätzlich Daten zur Körperstatik und zur Beweglichkeit des Patienten erfasst.

Bevorzugt sind die Daten zur Körperstatik und zur Beweglichkeit des Patienten, die in Schritt a) zusätzlich bestimmt werden, ausgewählt unter:
- Analyse der Ausrichtung und des Verlaufs der Wirbelsäule,
- Analyse der Beweglichkeit des Kopfes.

Die Erfassung von Daten zur Beweglichkeit des Kopfes und zum Verlauf der Wirbelsäule des Patienten erfolgt in erster Linie zu Dokumentationszwecken und zur Überprüfung der in dem erfindungsgemäßen Verfahren getroffenen Maßnahmen, z. B. zur Überprüfung des Verlaufs einer Therapie und/oder des Therapieerfolges.

Zur Analyse der Ausrichtung und des Verlaufs der Wirbelsäule können die im Stand der Technik bekannten Verfahren zur Oberflächenrasterung durch Auflichtscannen sowie haptische Verfahren eingesetzt werden. Bevorzugt werden hierzu haptische Verfahren, wie z. B. haptische Wirbelsäulen-Scanner eingesetzt, da diese Verfahren keine aufwändigen und teuren Apparaturen benötigen und die Qualität der erhaltenen Daten mit denen durch Auflichtscannen erhaltenen Daten vergleichbar ist.

Zur Erfassung bzw. Dokumentation der Beweglichkeit des Kopfes Patienten wird üblicherweise eine digitale Kamera eingesetzt, mit deren Hilfe die Bewegungen aufgenommen werden können. Die Beweglichkeit des Kopfes wird hierbei für alle drei Raumrichtungen, i.e. Linksdrehung Rechtsdrehung, Neigung nach oben und unten sowie die Neigung nach links und rechts, bestimmt. Dabei werden die Aufnahmen üblicherweise aus derselben Kameraposition durchgeführt, um die Aufnahmen mit vorangegangenen oder nachfolgenden Aufnahmen vergleichen zu können.

### Schritt b):

Ist eine ausbalancierte Körperstatik des Patienten in aufrechter gerader Position sichergestellt, erfolgt in einem nachfolgenden Schritt b) des erfindungsgemäßen Verfahrens die Erstellung eines biometrischen Fotos der Frontansicht des Kopfes des Patienten.

Zur Erstellung des biometrischen Fotos wird üblicherweise mit einer digitalen Kamera durchgeführt. Hierzu können prinzipiell alle digitalen Kameras eingesetzt werden, die sich für die Aufnahme biometrischer Fotos der Frontansicht eines Menschen eignen und Fotos zu erzeugen, die eine geringe Verzerrung aufweisen und sich naturgetreu skalierten lassen.

In der Regel wird in Schritt b) des erfindungsgemäßen Verfahrens nur ein biometrisches Foto der Frontansicht des Kopfes des Patienten erstellt. Bevorzugt handelt es sich hierbei um ein zweidimensionales Foto.

Bevorzugt werden zur Erstellung des biometrischen Fotos der Frontansicht des Kopfes des Patienten in Schritt b) eine Industriekamera eingesetzt. Bei Industriekameras handelt es sich um Kameras, die in der Industrie für messtechnische Aufgabenstellungen und zur Qualitätskontrolle eingesetzt werden. Die von diesen Kameras erzeugten Roh-Bilder werden anschließend mittels geeigneter Bildbearbeitungs-Software entzerrt und naturgetreu skaliert. Um den Grad der Verzerrung der Fotos möglichst gering zu halten, ist es vorteilhaft, die Industriekamera möglichst senkrecht zur Frontansicht des Kopfes des Patienten zu positioniert.

### Schritt c):

In einem nächsten Schritt des erfindungsgemäßen Verfahrens wird die therapeutischen Lage und Ausrichtung wenigstens eines anatomischen Symmetrieelements des Patienten anhand des in Schritt b) erstellen biometrischen Fotos unter Zuhilfenahme einer mathematischen Matrix berechnet. Die Berechnung erfolgt hierbei computergestützt. In einer vorteilhaften Ausführungsform ist die mathematische Matrix in eine Software zur Steuerung der einzelnen Verfahrensschritte integriert.

Die in Schritt c) des erfindungsgemäßen Verfahrens eingesetzte mathematische Matrix basiert im Wesentlichen auf
- der Lehre der Biometrik,
- Kenntnisse der menschlichen Anatomie,
- Kenntnisse mathematischer Gesetzmäßigkeiten im Zusammenhang mit dem Aufbau der menschlichen Anatomie, wie z. B. die Lehre des goldenen Schnittes,
- der mathematischen Lehre zu "Perfektoide Räume" und "Perfektoide Körper".

Bei der Lehre der Biometrik handelt es sich um die Anwendung mathematischer Methoden zur (zahlenmäßigen) Erfassung spezifischer biologischer Charakteristika. Die Biometrik stellt unter Anderem mathematische Verfahren zur Verfügung, welche die Objektivität und Vergleichbarkeit biologischer Daten, wie beispielsweise Daten spezifischer biologischer Charakteristika, sichern. Darüber hinaus prüft sie, ob die biologischen Daten signifikant sind und damit verallgemeinert werden können. Aus der Biometrie ist bekannt, dass die biometrischen Charakteristika, die sich beispielsweise aus der Frontansicht des Kopfes eines Menschen ermitteln lassen, wie beispielsweise der Pupillenabstand oder die Abweichung der Verbindungslinie der Pupillen von der Horizontalen, für jeden Menschen charakteristisch sind und Rückschlüsse auf dreidimensionale anatomische Strukturen des betreffenden Menschen ermöglichen (biometrische Fotoforensik). Die Erfassung biologischer Charakteristika aus biometrischen Fotos von menschlichen Gesichtern ist im Stand der Technik bekannt.

Bei den Kenntnissen der menschlichen Anatomie handelt es sich vorliegend um statistische Daten zum Aufbau des menschlichen Körpers, d. h. um statistisch gemittelte Werte empirisch bestimmter anatomischer Strukturen des menschlichen Körpers. Bevorzugt kommen in dem vorliegenden Verfahren Kenntnisse und Daten zum Aufbau des Schädels, insbesondere zum Aufbau und zur Anordnung der Kiefer und Kiefergelenke, und zum Aufbau und zur Ausrichtung der oberen Halswirbel des menschlichen Körpers zum Einsatz. Dem Fachmann sind solche Daten allgemein zugänglich. Z. B. kann der Fachmann hierzu auf im Stand der Technik bekannte einschlägige Literatur der menschlichen Anatomie und den darin enthaltenen Tabellenwerten, wie beispielsweise Tabellenwerte aus cephalometrischen Vermessungen, Baugut-Tabellen, gnatologische Normwerte, und Dergleichen, zurückgreifen.

Wie bereits eingangs ausgeführt, kann der anatomische Aufbau des menschlichen Körpers, aufgrund dessen Funktionalität und dessen bilateralen Aufbaus, zumindest bis zu einem gewissen Grad mathematisch beschrieben werden. So fand man bereits in der Antike heraus, dass anatomische Charakteristika des menschlichen Körpers, wie z. B. Abstände und die Größe anatomischer Strukturen des Gesichtes oder von skelettalen Strukturen des Menschen, oft in einem ganz bestimmten Verhältnis zueinander stehen, i.e. dem Verhältnis des goldenen Schnitts. Aus diesem Verhältnis lassen sich eine Vielzahl geometrischer Figuren, wie beispielsweise die Fibonacci-Spirale (Goldene-Spirale) oder das goldene Dreieck, ableiten, anhand derer sich die anatomischen Gegebenheiten des menschlichen Körpers recht gut beschreiben lassen. Darüber hinaus existieren weitere mathematische Gesetzmäßigkeiten, die sich aus der Vermessung skelettaler Strukturen, wie beispielsweise aus der Vermessung menschlicher Schädel, ergeben und zumindest für eine spezifische ethnische Bevölkerungsgruppe allgemeingültig sind. Diese allgemeingültigen mathematischen Gesetzmäßigkeiten sind dem Fachmann bekannt oder der Fachmann kann hierzu auf im Stand der Technik bekannte einschlägige Literatur des Aufbaus der menschlichen Anatomie zurückgreifen.

Bei der mathematischen Lehre zu "Perfektoide Räume" und "Perfektoide Körper" handelt es sich um eine von Prof. Peter Scholze entwickelte mathematische Theorie. Mit Hilfe dieser Theorie lassen sich Problemen in der arithmetischen algebraischen Geometrie lösen. Die hierbei von Scholze eingeführten Perfektoiden Körper dienen dazu, zahlentheoretische Probleme geometrisch begreifen zu können.

In der vorliegenden Erfindung werden die von dem Patienten erfassten biometrischen Daten mit allgemeingültigen anatomischen Werten und allgemeingültigen mathematischen Gesetzmäßigkeiten zum Aufbau des menschlichen Körpers zusammengeführt und auf deren Grundlage die optimale bzw. therapeutische Lage und Ausrichtung der anatomischen Symmetrieelemente des Patienten bestimmt. Bei diesem Vorgang handelt es sich um ein komplexes mathematisches Optimierungsproblem, bei dem die oben erwähnten mathematischen Methoden aus der Biometrik mit den oben dargelegten Kenntnissen zum Aufbau der menschlichen Anatomie in Einklang gebracht werden müssen. Dieses Optimierungsproblem lässt sich unter Zuhilfenahme der mathematischen Lehre zu "Perfektoide Räume" und "Perfektoide Körper" effizient und insbesondere mit sehr hoher Präzision lösen. Hierbei wird auf Ansätze aus der mathematischen Lehre zu "Perfektoide Körper" bzw. auf Teile der hierzu von Prof. Peter Scholze veröffentlichten Formeln zurückgegriffen. Diese Formeln lassen sich für praktische Anwendungen, wie das in dem erfindungsgemäßen Verfahren zu lösende Optimierungsproblem, nutzbar machen. Diese Nutzbarmachung kann von einem Fachmann der Mathematik mit tiefgreifenden Kenntnissen in der Algebra und der Zahlentheorie mit zumutbarem Aufwand bewerkstelligt werden.

Ohne die oben geschilderte Berechnungsmethode, i.e. ohne die Anwendung der mathematischen Lehre zu "Perfektoide Räume" und "Perfektoide Körper", könnten die relevanten anatomischen Bezugspunkte nur Näherungsweise, unter Heranziehung von Verallgemeinerungen, aus den in Schritt b) erhobenen biometrischen Daten ermittelt werden. Eine solche Näherung wäre wesentlich ungenauer und würde den Erfolg einer drauf basierenden Therapie des Patienten in Frage stellen.

Bei den in Schritt c) des erfindungsgemäßen Verfahrens berechneten anatomischen Symmetrieelementen handelt es sich üblicherweise um anatomische Symmetrieebenen, Kugeln, anatomische Symmetrieachsen und Gelenkachsen des Patienten. Bevorzugt ist das in Schritt c) berechnete wenigstens eine anatomischen Symmetrieelementen ausgewählt unter Kiefergelenkachse, Halswirbelachsen (Atlasachsen), Körpermittelachse (Mittelmeridian), Gaumenkugel, Rachenkugel, Kondylenkugel, Atlaskugel, Kauebene, Atlasebene, kraniale Symmetrieebene, Frontalebene, Saggitalebene und Transversalebene.

Besonders bevorzugt ist das in dem erfindungsgemäßen Verfahren berechnete wenigstens eine anatomischen Symmetrieelemente ausgewählt unter Kiefergelenkachse, Körpermittelachse (Mittelmeridian), Gaumenkugel, Rachenkugel, Kondylenkugel, Atlaskugel, Kauebene, Atlasebene.

Da sich die anatomischen Charakteristika von Menschen aus unterschiedlichen ethnischen Bevölkerungsgruppen signifikant unterscheiden können, müssen die in der mathematischen Matrix verwendeten Daten und Gesetzmäßigkeiten gegebenenfalls an die speziellen anatomischen Gegebenheiten des Patienten angepasst werden. Eine solche Anpassung kann problemlos bewerkstelligt werden. Hierzu werden die im Algorithmus der Matrix enthaltenen Daten zur menschlichen Anatomie, wie beispielsweise anatomische Längenverhältnisse, Abstände, Winkel, usw., sowie die in der Matrix verwendeten mathematischen Formeln zum Aufbau der menschlichen Anatomie durch die jeweils für die vorliegende ethnische Bevölkerungsgruppe geltenden anatomischen Daten und Formeln ersetzt.

In der Regel sind die in der mathematischen Matrix verwendeten Daten zur menschlichen Anatomie auf die in den westlichen Ländern am häufigsten vorkommenden ethnischen Bevölkerungsgruppen zugeschnitten, was in etwa einer 40-50 prozentigen Abdeckung der Weltbevölkerung entspricht.

Neben der mathematischen Matrix werden in Schritt c) des erfindungsgenäßen Verfahrens weitere Softwarebestandteile eingesetzt. Dabei handelt es sich üblicherweise um wenigstens eine Kamerasoftware und wenigstens eine Diagnosesoftware, sowie eine davon unabhängige Analysesoftware.

Die Kamerasoftware ist in der Regel direkt in der Kamera installiert und die Diagnosesoftware direkt in dem/den jeweiligen Diagnosegerät(en). Die Kamerasoftware dienen der Steuerung der Kamera und die Diagnosesoftware der Steuerung des/der jeweiligen Diagnosegerät(e), wie beispielsweise der Steuerung des haptischen Wirbelsäulen-Scanners.

Die Analysesoftware Steuert den computergestützten Schritt c) des erfindungsgemäßen Verfahrens. Die Analysesoftware enthält üblicherweise auch die zuvor beschriebene mathematische Matrix, mit deren Hilfe die Berechnungen der therapeutischen Lage und Ausrichtung der anatomischen Symmetrieelemente durchgeführt wird.

Bevorzugt umfasst der Schritt c) des erfindungsgemäßen Verfahrens die folgenden computergestützten Schritte:
c.1) Nachbearbeitung des in Schritt b) erstellten biometrischen Fotos,
c.2) Bestimmung der für die Berechnung der therapeutischen Lage und Ausrichtung wenigstens eines anatomischen Symmetrieelements des Patienten nötigen biometrischen Abstände und Winkel aus dem in Schritt c.1) nachbearbeiteten biometrischen Foto,
c.3) Übertragung der in Schritt c.2) bestimmten biometrischen Abstände und Winkel in eine mathematische Matrix, und
c.4) Berechnung der therapeutischen Lage und Ausrichtung wenigstens eines anatomischen Symmetrieelements des Patienten.

### Schritt c. 1):

In Schritt c.1) werden zunächst die in Schritt b) erstellten biometrischen Fotos nachbearbeitet.

Gegebenenfalls werden in Schritt c.1) zusätzliche diagnostische Daten, wie beispielsweise Wirbelsäulenscans, dreidimensionale Oberflächenscans der Mundhöhle bzw. der Zahnmorphologie und/oder Bilddaten zur Beweglichkeit des Kopfes, eingelesen und von der Analysesoftware gesichtet und aufbereitet.

Die Nachbearbeitung bzw. Aufbereitung der Daten in Schritt c.1) umfasst üblicherweise die nachfolgend aufgeführten computergestützten Operationen:

Die in Schritt b) erhaltenen biometrischen Fotos werden entzerrt, d. h. die durch die Kameraoptik hervorgerufenen Verzerrungen werden herausgerechnet.

Zusätzlich werden die in Schritt b) erhaltenen biometrischen Fotos auf einen Abbildungsmaßstab von 1:1 skaliert (naturgetreue Skalierung). Hierzu wird üblicherweise wenigstens ein Objekt mit bekannter Größe, wie beispielsweise ein Maßstab, in der Nähe des Kopfes des Patienten positioniert. Wie bereits zuvor erwähnt kann hierzu auch ein Lippenspreizer eingesetzt werden, der wenigstens einen Formkörper mit bekannter Größe enthält und/oder trägt. Mit Hilfe der so erhaltenen Distanzinformation(en) kann das Foto mit hoher Genauigkeit naturgetreu skaliert werden.

Die gegebenenfalls in Schritt c.1) zusätzlich eingelesenen dreidimensionalen diagnostischen Daten, wie beispielsweise dreidimensionale Oberflächenscans der Mundhöhle oder anderweitig gewonnene dreidimensionale Daten zur Zahnmorphologie, werden, falls erforderlich, in STL-Datensätze umgerechnet und anschließend in das softwareinterne Koordinatensystem transformiert.

### Schritt c.2):

Aus den in Schritt c.1) nachbearbeiteten Fotos, lassen sich anschließend Längen und Winkel anatomischer Charakteristika der Frontansicht des Kopfes des Patienten natur- und maßstabsgetreu und mit hoher Präzision herauslesen. Bevorzugt werden für die Berechnung der therapeutischen Lage und Ausrichtung wenigstens eines anatomischen Symmetrieelements des Patienten aus den nachbearbeiteten Fotos die Positionen der Mittelpunkte der Pupillen sowie der Pupillenabstand des Patienten bestimmt.

Die Bestimmung der exakten Positionen der Mittelpunkte der Pupillen des Patienten kann dabei automatisch durch die Bildbearbeitungssoftware erfolgen und/oder von Hand durch den behandelnden Arzt oder durch eine mit der Auswertung betrauten externen Person mit entsprechenden Fachkenntnissen bestimmt werden. Bevorzugt erfolgt die Bestimmung der exakten Positionen der Mittelpunkte der Pupillen des Patienten entweder durch die Bildbearbeitungssoftware, welche die Positionen der Pupillen zunächst grob festlegt, bevor diese von einer mit der Auswertung betrauten Person mit entsprechenden Fachkenntnissen manuell nachkorrigiert werden, oder ausschließlich von Hand durch die mit der Auswertung betrauten Person.

### Schritt c.3):

Die anhand der biometrischen Fotos ermittelten anatomischen Werte sowie die gegebenenfalls zusätzlich eingelesenen dreidimensionalen diagnostischen Daten des Patienten werden anschließend in die mathematische Matrix übertragen. Die mathematische Matrix kann in der Analysesoftware eingebettet sein aber auch unabhängig von der Analysesoftware als eigenständiges Programm operieren. Die Übertragung der Werte und Daten erfolgt üblicherweise automatisch, i.e. in einem computergestützten Arbeitsgang.

Die Übertragung erfolgt entweder vor Ort, beispielsweise auf einem Computer, auf dem die Analysesoftware bzw. die Matrix gespeichert ist und abgespielt werden kann, oder die Rohdaten, i.e. die biometrischen Fotos sowie die gegebenenfalls zusätzlich eingelesenen dreidimensionalen diagnostischen Daten des Patienten, werden verschlüsselt an ein hierfür entsprechend eingerichtetes externes Analysezentrum übermittelt unter Verwendung gängiger Übermittlungsverfahren. Bevorzugt werden die Rohdaten an ein externes Analysezentrum übermittelt. Bevorzugt werden auch die zuvor unter Schritt c.2) beschriebene Ermittlung der relevanten anatomischen Werte aus den biometrischen Fotos sowie die nachfolgend unter Schritt c.4) beschriebenen Berechnungen durch ein hierfür entsprechend eingerichtetes externes Analysezentrum durchgeführt.

### Schritt c.4):

Die Mathematische Matrix errechnet anschließend die therapeutische Lage und Ausrichtung wenigstens eines anatomischen Symmetrieelements des Patienten. Bei diesen Symmetrieelementen handelt es sich um die zuvor genannten.

Die Berechnung der genannten anatomischen Symmetrieelemente erfolgt dabei bevorzugt nach folgendem Schema.

Aus den in Schritt c.2) ermittelten biometrischen Daten wird mit Hilfe der mathematischen Matrix basierend auf den zuvor genannten Kenntnissen und Daten zur Anatomie des Menschen zunächst die patientenindividuelle Pupillenneigung (Winkel der Abweichung der Verbindungslinie der Pupillen zur Horizontalen) sowie die Größe und Lage der Gaumenkugel zu allen relevanten skelettalen Strukturen sowie zur ermittelten aufrechten geraden Körperhaltung des Patienten berechnet. Die Berechnung der Gaumenkugel beinhaltet gleichzeitig auch die Berechnung des Mittelmeridians, der cranialen Symmetrieachse und der cranialen Symmetrieebenen.

Anschließend wird die Gaumenkugel in dreidimensionale Ebenen zerlegt, d. h. es wird ein dreidimensionales Raster von Ebenen erzeugt, die senkrecht und/oder parallel zum ermittelten Mittelmeridian stehen. Zusätzlich werden passend zu den bereits bestimmten anatomischen Ebenen und Strukturen, unter anderem die Rachenkugel, die Kondylenkugeln und die Atlaskugel berechnet.

In einem weiteren Schritt werden die therapeutische Lage der Kauebene sowie der Rotationsachse des Unterkiefers berechnet.

Die so ermittelte therapeutische Lage der Kauebene und der Rotationsachse des Unterkiefers ermöglicht nun beispielsweise die exakte patientenindividuelle therapeutische Zuordnung des Ober- und Unterkiefers des Patienten zueinander. Diese Zuordnung erfolgt hierbei ausschließlich auf rechnerischem Wege. Aus der lagerichtigen/therapeutischen Zuordnung der Ober- und Unterkiefer können auch deren Vertikaldimensionen nun exakt berechnet werden.

In einer bevorzugten Ausführungsform des erfindungsgemäßen Verfahrens umfasst die Analysesoftware, zusätzlich zur Bestimmung der therapeutischen Lage und Ausrichtung wenigstens eines der zuvor genannten anatomischen Symmetrieelemente des Patienten, wenigstens eine der nachfolgenden computergestützten Berechnungsschritte:
- Berechnung von STL-Datensätzen der patientenindividuell und lagerichtig zugeordneten Ober- und Unterkiefermodelle des Patienten;
- Berechnung von STL-Datensätzen für Bauteile zur patientenindividuellen Modifikation eines handelsüblichen Artikulators;
- Berechnung von STL-Datensätzen von patientenindividuellen Orthesen;
- Berechnung von STL-Datensätzen von Waxup Strukturen;
- Berechnung von Hilfsebenen und Hilfspunkte für die Erstellung von Zahnprothetik und/oder zur Planung von Implantaten;
- Berechnung von Zahnbögen sowie die Größe und Lage der Zähne;
- Erstellung einer umfangreichen Patientendokumentation.

Die Analysesoftware berechnet üblicherweise in einem ersten Schritt die therapeutische Lage der eingelesenen Ober- und Unterkiefermodelle relativ zu den berechneten Symmetrieelementen, insbesondere zur berechneten Kiefergelenksachse und Kauebene. Die Analysesoftware erstellt anschließen STL-Datensätze der patientenindividuell und lagerichtig zugeordneten Modelle der Ober- und Unterkiefer des Patienten.

Auf der Grundlage der so lagerichtig zugeordneten Modelle der Ober- und Unterkiefer des Patienten können weitere Berechnungen, wie oben erwähnt, durchgeführt werden.

Beispielsweise kann die Analysesoftware zusätzlich Bauteile zur patientenindividuellen Modifikation eines handelsüblichen Artikulators berechnen, mit deren Hilfe sich die Ober- die Unterkiefermodelle patientenindividuell und lagerichtig, i.e. therapeutisch, in diesen Artikulatoren ausrichten (artikulieren) lassen. In diesem Zusammenhang bedeutet der Ausdruck "therapeutisch ausrichten", dass die Ober- und Unterkiefermodelle in den Artikulatoren derart positioniert werden, dass die Scharnierachse des Artikulators nun der berechneten therapeutischen patientenindividuellen Kiefergelenksachse des Patienten entspricht und dass die so erzeugte Bissebene der berechneten therapeutischen Bissebene entspricht. Zur therapeutischen Ausrichtung wird die berechnete therapeutische patientenindividuelle Kiefergelenksachse virtuell über die feste Achse eines digitalen dreidimensionalen Modells des betreffenden Artikulators gelegt und die Lage der Ober- und Unterkiefermodelle zurückgerechnet.

Bei den von der Analysesoftware berechneten Orthesen handelt es sich beispielsweise um Aufbissschienen zur Behandlung von funktionellen Störungen des Kauorgans, Kraftschienen zum Lösen oder Verhindern von Verkrampfungen bzw. übermäßigen Anspannungen der Muskulatur, insbesondere der Kau- und Nackenmuskulatur, während der Ausübung sportlicher Tätigkeiten, oder um Knirscherschienen.

Bei den von der Analysesoftware berechneten Waxup-Strukturen handelt es sich üblicherweise um Modelle welche die zukünftige Zahnrestauration, wie beispielsweise Zahnaufbauten oder Zahnersatz, darstellen.

Bei den von der Analysesoftware berechneten Hilfsebenen und Hilfspunkten für die Erstellung von Zahnprothetik und/oder zur Planung von Implantaten handelt es sich üblicherweise um anatomische Ebenen und/oder Punkte, welche die Ausrichtung der zu restaurierenden Zahnreihe(n) relativ zur ermittelten therapeutischen Kauebene und zur ermittelten therapeutischen Kiefergelenksachse festlegen. Zusammen mit der ebenfalls von der Analysesoftware berechenbaren Größe und Form der zu restaurierenden Zahnbögen sowie die Größe und Lage der Zähne in diesen Zahnbögen liefert das Verfahren wichtige therapeutische Informationen für die Planung von Zahnersatz.

Die von der Analysesoftware erstellte Patientendokumentation enthält alle ermittelten diagnostischen Daten des Patienten über den gesamten Therapieverlauf und wird automatisch generiert und aktualisiert.

Das erfindungsgemäße Verfahren ermöglicht die Bestimmung der therapeutischen Lage und Ausrichtung weitgehend aller für die Körperhaltung des Patienten relevanten anatomischen Symmetrieelemente. Daher kann das erfindungsgemäße Verfahren in verschiedenen medizinischen Bereichen eingesetzt werden, wie beispielsweise in der Orthopädie, der Kieferorthopädie, der Osteopathie, der Rehabilitationsmedizin oder der Sportmedizin, zur Behandlung unterschiedlichster Erkrankungen, bei denen Haltungsfehler bzw. eine unausgeglichene Körperstatik eine Rolle spiele.

Das erfindungsgemäße Verfahren eignet sich beispielsweise zur Planung patientenindividueller Prothesen und Orthesen, zur Planung und Überprüfung von Therapiemaßnahmen im Zusammenhang mit Fehlbelastungen und/oder Haltungsschäden bei Kindern und Erwachsenen, zur Planung und Überprüfung von Rehabilitationsmaßnahmen oder zur Optimierung von Bewegungsabläufen im Leistungssport. So ermöglicht das Verfahren die Herstellung sogenannter Kraftschienen, welche die Kiefer während einer sportlichen Betätigung in einer entspannten physiologischen Lage halten, was zu einer signifikanten Verbesserung der Kraftentwicklung und Beweglichkeit führen kann.

Das erfindungsgemäße Verfahren eignet sich in vorteilhafter Weise für die Herstellung patientenindividueller Aufbissschienen, für die Planung und Berechnung von Zahnprothetik und für die patientenindividuelle Modifikation von Artikulatoren.

### Aufbissschienen:

Eine Ausführungsform des erfindungsgemäßen Verfahrens betrifft daher ein Verfahren zur Herstellung von Aufbissschienen zur Verwendung in der Behandlung eines Patienten mit wenigstens einer Erkrankung, die von einer funktionellen Störung des Kauorgans ausgeht, umfassend die folgenden Schritte:
i) computergestützte Bestimmung der therapeutischen Lage und Ausrichtung wenigstens eines für die Stellung der Kiefer relevanten anatomischen Symmetrieelements des Patienten wie zuvor und im Folgenden definiert,
ii) computergestützte lagerichtige Zuordnung digitalisierter Ober- und Unterkiefermodelle zu den in Schritt i) bestimmten Symmetrieelementen, und
iii) Erzeugung wenigstens eines STL-Datensatzes der Aufbissschiene.

Patienten mit Erkrankungen des Kauorgans stellen einen wesentlichen Bestandteil der zahnärztlichen Behandlungen, insbesondere kieferorthopädischen Behandlungen, dar. Ein Kiefergelenk besteht im Wesentlichen aus einem Gelenkkopf (Condylus mit Caput mandibulae) des Unterkiefers, der beweglich in einer Gelenkgrube (Fossa articularis) des Schläfenbeins angeordnet ist. Die Ausrichtung des Kiefergelenks und die damit verbundene Kaumuskellage sind wesentlich an der Kopfhaltung beteiligt. Die Kopfhaltung hat wiederum maßgeblichen Einfluss auf die gesamte Körperhaltung. Dadurch entstehen häufig Wechselwirkungen der Kaumuskellage mit dem Halteapparat im Nackenbereich während dieser wiederum mit der gesamten Körperstatik interagiert. Umgekehrt hat die Körperhaltung bzw. die Körperstatik einen wesentlichen Einfluss auf die Stellung der Kiefergelenke bzw. der Kaumuskellage.

Schlüsselpunkte in diese Wechselwirkung sind die Halswirbelsäule, insbesondere der Atlas-Halswirbel, der Schultergürtel sowie das Becken.

Eine häufige Ursache von Erkrankungen, die von einer funktionellen Störung des Kauorgans ausgehen, ist eine pathologische und damit nicht physiologische Lage des Kiefergelenkkopfes in der Kiefergelenkgrube und eine damit verbundene Über- oder Fehlbelastung der gesamten beteiligten anatomischen Strukturen, wie Bänder, Knorpel, Muskulatur, Zähne und der Kiefergelenke. Dies führt in der Regel zu schmerzhaften Verspannungen der Kaumuskulatur bis hin zu dauerhafter Schädigung von Bändern, Sehnen und Knorpel sowie zur Destruktion beteiligter knöcherner Strukturen. Zu den Symptomen solcher Erkrankungen gehören Kopf-, Ohren- oder Gesichtsschmerzen sowie das nächtliche Pressen und Knirschen der Zähne. Daneben können weitere Beschwerden, die nicht direkt mit dem Kausystem in Verbindung stehen, wie Tinnitus, Sehstörungen, Schlafstörungen oder Gleichgewichtsstörungen und dergleichen oder Probleme im Bereich der Wirbelsäule sowie des gesamten Bewegungsapparates, z. B. der Hüfte, der Beine, der Knie, der Füße und des Schulterbereichs, auftreten.

Zur Behandlung von Erkrankungen, die von einer funktionellen Störung des Kauorgans ausgehen, werden häufig Aufbissschienen, nachfolgend auch als Schiene oder Zahnschiene bezeichnet, eingesetzt. Ziel des Einsatzes solcher Aufbissschienen ist es, den Patienten von einer pathologischen Okklusion und/oder Kaubewegung abzuhalten bzw. ihm diese abzugewöhnen. In der Folge soll die physiologische Zentrik der Gelenkköpfe in den Gelenkgruben des Kiefergelenks wiederhergestellt werden. Damit soll die Über- und Fehlbelastung der Zähne und Kiefergelenke zumindest teilweise beseitigt werden, was in der Regel zu einer verbesserten Positionierung der Kiefergelenke und einer Relaxierung der Kiefermuskulatur führt.

Die hier verwendeten Begriffe "Aufbissschiene" bezeichnet ein Mittel zum Aufbeißen für den Oberkiefer und/oder Unterkiefer, der wenigstens einen Aufnahmebereich zur Aufnahme der Zähne des Oberkiefers und/oder Unterkiefers und/oder wenigstens einen Kontaktbereich zum Kontaktieren der Zähne des Oberkiefers und/oder des Unterkiefers aufweist.

Der Aufnahmebereich kann zusätzlich Mittel zum Führen der Zähne aufweisen. Bei dem Mittel zum Führen der Zähne kann es sich beispielsweise um eine Frontzahnführung, Eckzahnführung, Gruppenführung, balancierte Okklusionen oder Seitenzahnführung sowie um Kombinationen davon handeln.

Der Aufnahmebereich dieser Aufbissschienen und Folge-Aufbissschienen ist so profiliert, dass ein ausreichend sicherer Halt der Schienen auch bei Belastung (z. B. Beißen, Sprechen, Bruxismus) gewährleistet ist. Gegebenenfalls kann der Halt der Schienen mit üblichen Haltemitteln (z. B. Knopfanker) verbessert werden.

Der Kontaktbereich wiederum kann entweder nicht profiliert, d. h. nicht an die jeweilige Zahnmorphologie (Kauprofil) des Patienten angepasst sein, sodass eine vollständige Bewegungsfreiheit der Kiefer zueinander in der maximalen Kontaktposition bestehen bleibt, oder geringfügig profiliert, d. h. geringfügig oder grob an die jeweilige Zahnmorphologie des Patienten angepasst sein, sodass die Bewegungsfreiheit der Kiefer zueinander in der End-Bissstellung eingeschränkt wird.

Die in diesem Zusammenhang verwendeten Ausdrücke "geringfügig profiliert" bzw. "geringfügig oder grob an die Zahnmorphologie des Patienten angepasst" bedeuten, dass die Profilierung keine formschlüssige Negativabbildung (Abdruck) der Kontaktbereiche der Zähne mit der Aufbissschiene darstellt, sondern dass die Profilierung aus zur Oberfläche der Aufbissschiene hin erweiterten Vertiefungen bzw. Gruben und/oder Rampen im Kontaktbereich der Zähne besteht (okklusale Impressionen). Anders ausgedrückt handelt es sich hierbei um geglättete Abdrücke der Kontaktbereiche der Zähne. Die Glättung der Kontaktbereiche kann mehr oder weniger stark ausgeprägt sein, je nachdem wie groß die gewünschte Bewegungsfreiheit der Kiefer in der End-Bissstellung sein soll.

Sinngemäß bedeuten die Ausdrücke "stark profiliert" bzw. "vollständig an die Zahnmorphologie des Patienten angepasst", dass die Profilierung eine im Wesentlichen formschlüssige Negativabbildung (Abdruck) des Kontaktbereichs der Zähne des Patienten darstellt, welches die Bewegungsfreiheit der Kiefer in der End-Bissstellung stark einschränkt oder sogar vollständig unterbindet.

Der wenigstens eine Aufnahmebereich zur Aufnahme der Zähne des Oberkiefers und/oder Unterkiefers weist bevorzugt eine geringe bis starke Profilierung auf. Besonders bevorzugt ist der wenigstens eine Aufnahmebereich stark profiliert, d. h. vollständig an die jeweilige Zahnmorphologie (Kauprofil) des Patienten angepasst.

Die Stärke der Aufbissschiene bzw. der Folge-Aufbissschiene, d. h. der Abstand des Aufnahmebereichs oder Kontaktbereichs für den ersten Kiefer zum Aufnahmebereich oder Kontaktbereich für den dem ersten Kiefer gegenüberliegend angeordneten zweiten Kiefer, kann stark variieren und orientiert sich an der therapeutischen Bissebene und der ermittelten Ruheschwebelage der Kiefer im entspannten Zustand, gegebenenfalls mit Lageveränderung durch z. B. Protrusion und/oder Laterotrusion.

### Schritt i):

Schritt i) dieser Ausführungsform des erfindungsgemäßen Verfahrens umfasst die computergestützte Bestimmung der therapeutischen Lage und Ausrichtung wenigstens eines für die Stellung der Kiefer relevanten anatomischen Symmetrieelements des Patienten.

Bei den für die Stellung der Kiefer relevanten anatomischen Symmetrieelementen des Patienten handelt es sich um die zuvor erwähnten Symmetrieelemente. Die Bestimmung der therapeutischen Lage und Ausrichtung dieser Symmetrieelemente erfolgt ebenfalls wie zuvor beschrieben.

### Schritt ii):

In Schritt ii) werden digitalisierte Ober- und Unterkiefermodelle lagerichtig zu den in Schritt i) bestimmten Symmetrieelementen zugeordnet.

Hierbei kann entweder auf bereits bestehende digitalisierte Ober- und Unterkiefermodelle zurückgegriffen werden oder es werden neue digitalisierte Ober- und Unterkiefermodelle des Patienten erstellt.

Zur Erstellung der digitalisierten Ober- und Unterkiefermodelle eignen sich prinzipiell alle im Stand der Technik bekannten Verfahren zur dreidimensionalen Erfassung der Oberfläche anatomischer Strukturen oder Abdrücke anatomischer Strukturen, die mittels Computer-Programmen eingelesen, bildlich bzw. graphisch dargestellt und bearbeitet werden können. Bevorzugt werden die digitalisierten Ober- und Unterkiefermodelle mittels Kernspintomographie bzw. Magnetresonanztomographie (MRT) oder Oberflächenrasterung durch Auflichtscannen, beispielsweise mit Hilfe von Auflichtscannern, erstellt.

Diese bestehenden oder neu erstellten digitalisierten Ober- und Unterkiefermodelle werden lagerichtig zu den in Schritt i) bestimmten Symmetrieelementen zugeordnet. Diese Zuordnung erfolgt computergestützt, d. h. die therapeutische Lage der Ober- und Unterkiefermodelle wird berechnet, wie bereit zuvor beschrieben.

### Schritt iii):

In einem nachfolgenden Schritt werden STL-Datensätze der Aufbissschienen erzeugt. Hierzu wird zunächst anhand der lagerichtig zugeordneten Ober- und Unterkiefermodelle sowie dem ermittelten Abstand bzw. Öffnungswinkel der Kiefer im entspannten Zustand ein virtuelles Modell der Aufbissschiene berechnet. Selbstverständlich lassen sich bei Bedarf auch mehrere virtuelle Modelle von Aufbissschienen für unterschiedliche Öffnungswinkel der lagerichtig zugeordneten Ober- und Unterkiefermodelle erstellen.

Von diesen Modellen werden anschließend STL-Datensätze generiert.

Die Ausgabe der Aufbissschienen erfolgt bevorzugt mit 3D formgebenden Geräten, wie z. B. 3D-Druckern und 3D-Fräseinheiten.

Die Behandlung eines Patienten mit wenigstens einer Erkrankung, die von einer funktionellen Störung des Kauorgans ausgeht, erfolgt in der Regel über einen längeren Zeitraum, z. B. über einen Zeitraum von einigen Monaten bis mehreren Jahren, z. B. über einen Zeitraum von 4 Monaten bis 5 Jahren. Während dieser Zeit können Umstände eintreten, die eine Anpassung der Aufbissschiene erforderlich machen. Beispielsweise können sich anatomische Gegebenheiten, die einen Einfluss auf die Kieferstellung und/oder Kopf- und Körperhaltung haben, durch Krankheit, Unfall und/oder durch Operationen ändern.

Daneben ist es vorteilhaft, den Behandlungsfortschritt in regelmäßigen Abständen zu überprüfen.

Aus diesem Grund werden in einer bevorzugten Ausführungsform die Schritte i) bis iii) einmal oder mehrmals, z. B. 1- bis 10-mal, wiederholt.

### Vorrichtung:

Ein weiterer Gegenstand der Erfindung betrifft eine Vorrichtung zur Sicherstellung einer ausbalancierten Körperstatik des Patienten in aufrechter gerader Position und zur Erstellung eines biometrischen Fotos der Vorderansicht des Kopfes eines Patienten, umfassend:
- eine erste digitale Kamera zur Aufnahme eines biometrischen Fotos der Frontansicht des Kopfes des Patienten in aufrechter gerader Position,
- ein Mittel zum Ausgleich der Längendifferenz der Beine des Patienten,
- wenigstens ein Mittel zum Auffinden einer ausbalancierten Körperstatik des Patienten, und
- wenigstens ein Mittel zur waagerechten Ausrichtung der Vorrichtung.

Bei der in der Vorrichtung enthaltenen ersten digitalen Kamera zur Aufnahme eines biometrischen Fotos der Frontansicht des Kopfes des Patienten handelt es sich bevorzugt um eine Industriekamera, wie zuvor definiert.

In der erfindungsgemäßen Vorrichtung wird die Kamera üblicherweise vor den Patienten Positioniert. Wie bereits oben erwähnt ist es vorteilhaft, die Industriekamera möglichst senkrecht zur Frontansicht des Kopfes des Patienten zu positioniert, um keine unnötigen Verzerrungen zu generieren. Damit die Kamera entsprechend positioniert werden kann, ist diese bevorzugt an einem Stativ, insbesondere an einem höhenverstellbaren Stativ, befestigt.

Bei dem Mittel zum Ausgleich der Längendifferenz der Beine des Patienten handelt es sich, wie bereits zuvor ausgeführt, üblicherweise um eine Standunterlage, auf die sich der Patient in aufrechter gerader Position stellt, und welche eine allfällige unterschiedliche Längendifferenz der Beine ausgleichen kann.

Insbesondere wird in der erfindungsgemäßen Vorrichtung als Mittel zum Ausgleich der Längendifferenz der Beine des Patienten eine viskoelastische Standunterlage eingesetzt, die zuvor und im Folgenden auch als "Balance-Pad" bezeichnet wird.

Als Mittel zum Auffinden einer ausbalancierten Körperstatik des Patienten eignen sich üblicherweise alle im Stand der Technik bekannte Mittel zum Auffinden einer senkrechten Ausrichtung eines Gegenstandes (Ermittlung der Lotrechten oder Senkrechten). Üblicherweise handelt es sich hierbei um Mittel, die den Verlauf der Senkrechten durch wenigstens eine Linie kennzeichnen.

Bevorzugt wird das wenigstens eine Mittel zum Auffinden einer ausbalancierten Körperstatik des Patienten an einem Stativ, insbesondere an einem höhenverstellbaren Stativ, angebracht. Besonders bevorzugt wird das wenigstens eine Mittel zum Auffinden einer ausbalancierten Körperstatik des Patienten an demselben höhenverstellbaren Stativ angebracht, an dem auch die erste digitale Kamera befestigt ist.

Bevorzugt handelt es sich bei dem wenigstens einen Mittel zum Auffinden einer ausbalancierten Körperstatik des Patienten um wenigstens ein Lot. Bevorzugt ist das Lot ausgewählt unter einem Schnurlot, einem optischen Lot oder einem Laserlot. Insbesondere wird als Lot ein Schnurlot eingesetzt.

In einer bevorzugten Ausführungsform der erfindungsgemäßen Vorrichtung wird als zusätzliches Mittel zum Auffinden der ausbalancierten Körperstatik des Patienten ein Einwegspiegel zwischen der ersten digitalen Kamera und dem Patienten positioniert. Wie bereits oben ausgeführt, ermöglicht diese zusätzliche Orientierungshilfe es dem Patienten seine Körperhaltung selbstständig auszurichten und eine aufrechte gerade Körperhaltung, bei der sich die Körperstatik in einem ausbalancierten Zustand befindet, selbstständig aufzufinden. Die selbständige Auffindung dieser ausbalancierten Körperstatik ist noch effektiver, wenn das wenigstes eine Lot direkt vor dem Einwegspiegel positioniert wird.

In einer besonders bevorzugten Ausführungsform der Vorrichtung ist das wenigstens eine Mittel zum Auffinden der ausbalancierten Körperstatik des Patienten ausgewählt aus einem Lot und ein Einwegspiegel, wobei der Einwegspiegel zwischen der ersten digitalen Kamera und dem Patienten positioniert wird, und das Lot zwischen dem Einwegspiegel und dem Patienten, insbesondere unmittelbar vor dem Einwegspiegel, positioniert wird.

Wie zuvor erwähnt sollte zur Sicherstellung einer ausbalancierten Körperstatik des Patienten der Untergrund, auf dem der Patient steht, möglichst waagerecht ausgerichtet sein.

Bei dem wenigstens einen Mittel zur waagerechten Ausrichtung der Vorrichtung handelt es sich um die zuvor genannten Mittel. Bevorzugt handelt es sich hierbei um eine waagerecht ausrichtbare Bodenplatte, auf die sich der Patient in aufrechter gerader Position stellt. Falls gewünscht, kann die Größe der Bodenplatte so gewählt werden, dass auch die übrigen Mittel der Vorrichtung auf der waagerecht ausrichtbaren Bodenplatte positioniert werden können.

Gegebenenfalls kann die Vorrichtung zusätzlich zu den oben beschriebenen Mitteln eine oder mehrere der folgenden Mittel umfassen:
- Wirbelsäulen-Scanner zur Erfassung der Ausrichtung und des Verlaufs der Wirbelsäule des Patienten,
- eine zweite digitale Kamera auf einem Stativ zur Erfassung der Kopfbeweglichkeit des Patienten,
- eine Orientierungshilfe für die waagerechte Ausrichtung des Unterkiefers.

Der Wirbelsäulen-Scanner dient der Analyse der Ausrichtung und des Verlaufs der Wirbelsäule des Patienten. Hierzu könne die im Stand der Technik bekannten Wirbelsäulen-Scanner eingesetzt werden. Bevorzugt handelt es sich hierbei um haptische Wirbelsäulen-Scanner. Die Analyse der Ausrichtung und des Verlaufs der Wirbelsäule des Patienten dient üblicherweise zur Dokumentation des Therapieverlaufs und/oder des Therapieerfolges.

Als zweite digitale Kamera zur Erfassung der Kopfbeweglichkeit des Patienten können alle handelsüblichen Digitalkameras eingesetzt werden. Die zweite digitale Kamera wird üblicherweise an einem Stativ befestigt.

Wie bereits oben ausgeführt, handelt es sich bei der Orientierungshilfe für die waagerechte Ausrichtung des Unterkiefers üblicherweise um ein Stativ, insbesondere um ein höhenverstellbares Stativ, mit einer daran befestigten Kinnstütze. Die Kinnstützte kann bei Bedarf den Patienten dabei unterstützen den Unterkiefer waagerecht auszurichten, indem er die Kinnstütze mit der Unterseite des Kinns berührt.

Die digitale Kamera, das Lot, der Einwegspiegel, sowie, falls vorhanden, die Orientierungshilfe für die waagerechte Ausrichtung des Unterkiefers und/oder die zweite digitale Kamera, sind üblicherweise an einer geeigneten Befestigung angebracht. Die Befestigung ist bevorzugt ausgewählt aus einer Wand einem Gestell oder einem Stativ mit einer Basisplatte. Da die erfindungsgemäße Vorrichtung bevorzugt mobil einsetzbar sein soll, ist die Befestigung besonders bevorzugt ausgewählt unter einem Gestell oder einem Stativ mit einer Basisplatte. Ganz besonders bevorzugt ist die digitale Kamera, das Lot, der Einwegspiegel, sowie, falls vorhanden, die Orientierungshilfe für die waagerechte Ausrichtung des Unterkiefers und/oder die zweite digitale Kamera, an einem Stativ mit Basisplatte, insbesondere an einem höhenverstellbaren Stativ mit Basisplatte angebracht.

Die erfindungsgemäße Vorrichtung lässt sich in vorteilhafter Weise in dem zuvor beschriebenen erfindungsgemäßen Verfahren einsetzen und wurde insbesondere für die Durchführung des erfindungsgemäßen Verfahrens entwickelt.

Ein weiterer Gegenstand der vorliegenden Erfindung betrifft somit eine Vorrichtung wie zuvor und im Folgenden definiert zur Verwendung in einem Verfahren wie zuvor und im Folgenden definiert.

### Bausatz:

Ein weiterer Gegenstand der vorliegenden Erfindung betrifft einen Bausatz für die patientenindividuelle Modifikation eines konfektionierten (handelsüblichen) Artikulators, umfassend wenigstens ein Bauteil zur Fixierung des therapeutischen Abstandes und der therapeutischen Ausrichtung der Kiefermodelle zur Scharnierachse des Artikulators.

Bei der Verwendung eines handelsüblichen Artikulators werden Modelle der Ober- und Unterkiefer des Patienten in die hierfür vorgesehenen Halterungen am Artikulator eingesetzt bzw. eingeklebt. Herbei besteht die Schwierigkeit, die Ober- und Unterkiefermodelle im Artikulator so auszurichten, dass diese die anatomischen Gegebenheiten des Patienten möglichst gut wiedergeben. Im Stand der Technik werden hierfür aufwändige Verfahren zur Übertragung anatomischer Parameter, die direkt am Patienten, beispielsweise mit Hilfe eines Gesichtsbogens, gemessen werden, in den Artikulator eingesetzt. Noch schwieriger gestaltet sich die Positionierung der Ober- und Unterkiefermodelle im Artikulator, wenn deren Ausrichtung den für den Patienten ermittelten therapeutischen Lagen der Ober- und Unterkiefer (therapeutische Soll-Lagen) und nicht lediglich den momentanen Lagen der Ober- und Unterkiefer (Ist-Lagen) entsprechen soll.

Die vorliegende Erfindung ermöglicht es, Bauteile in Form eines Bausatzes für handelsübliche Artikulatoren zu generieren, mit deren Hilfe die patientenindividuelle therapeutische Lage der Ober- und Unterkiefermodelle in dem betreffenden Artikulator relativ zur Scharnierachse des Artikulators mit hoher Präzision therapeutisch ausgerichtet und in dieser Lage fixiert werden können.

Abhängig von der Konstruktion des Artikulators, für den die Bauteile bestimmt sind, und dem gewählten Herstellverfahren, mit dem die Bauteile erzeugt werden sollen, können die Bauteile des erfindungsgemäßen Bausatzes einheitliche Formkörper sein oder aus zwei oder mehr als zwei Teilen bestehen. In letzterem Fall werden zwei oder mehr als zwei Arten von Bauteilen bereitgestellt.

In einer ersten Ausführungsform besteht der Bausatz aus einem Bauteil, das aus einem einheitlichen Formkörper besteht. Das Bauteil dieser ersten Ausführungsform legt somit sowohl den therapeutischen Abstand der Kiefermodelle zur Scharnierachse des Artikulators als auch die räumliche Ausrichtung der Kiefermodelle (Rotation entlang der X-, Y- und Z-Achse) relativ zur Scharnierachse des Artikulators fest.

Zur Berechnung der Form des Bauteils wird zunächst, ausgehend von den durch das oben beschriebene erfindungsgemäße Verfahren ermittelten relevanten Symmetrieelementen, die therapeutische Lage der Ober- und Unterkiefermodelle bestimmt. Anschließend wird, wie bereits oben ausgeführt, die therapeutische patientenindividuelle Kiefergelenksachse virtuell über die feste Achse eines digitalen dreidimensionalen Modells des Artikulators gelegt und die Lage der Ober- und Unterkiefermodelle im Artikulator zurückgerechnet. Anhand der so festgestellten therapeutischen Lage der Ober- und Unterkiefermodelle im Artikulator wird wiederum ein virtuelles Modell des Bauteils berechnet aus diesem dann, falls gewünscht, ein entsprechender STL-Datensatz generiert wird.

In einer zweiten Ausführungsform besteht der Bausatz aus zwei Arten von Bauteilen, i.e. (einem) Bauteil(e) zur Fixierung des therapeutischen Abstandes der Kiefermodelle zur Scharnierachse des Artikulators und (einem) Bauteil(e) zur Fixierung der therapeutischen Ausrichtung der Kiefermodelle zur Scharnierachse des Artikulators.

Der Gegenstand dieser zweiten Ausführungsform betrifft daher einen Bausatz für die patientenindividuelle Modifikation eines konfektionierten Artikulators, umfassend:
- wenigstens ein Bauteil zur Fixierung des therapeutischen Abstandes der Kiefermodelle zur Scharnierachse des Artikulators,
- wenigstens ein Bauteil zur Fixierung der therapeutischen Ausrichtung der Kiefermodelle zur Scharnierachse des Artikulators.

Die erste Art von Bauteilen legt den therapeutischen Abstand bzw. die therapeutische Position der Kiefermodelle zur Scharnierachse des Artikulators fest. Die zweite Art von Bauteilen legt die räumliche Ausrichtung der Kiefermodelle (Rotation entlang der X-, Y- und Z-Achse) relativ zur Scharnierachse des Artikulators fest.

Die Form der Bauteile dieser zweiten Ausführungsform wird analog zur ersten Ausführungsform berechnet. Aus den berechneten virtuellen Modellen der Bauteile werden dann entsprechende STL-Datensätze generiert.

Da die Bauteile dieser zweiten Ausführungsform in zwei Bauteilarten aufgeteilt werden, ist deren Form sehr einfach. Die Bauteile lassen sich daher in vorteilhafter Weise mit Hilfe von 3D formgebenden Geräten, wie z. B. 3D-Druckern oder 3D-Fräseinheiten, erzeugen.

Selbstverständlich können die oben beschriebenen Bauteile auch aus mehr als zwei Teilen bestehen, falls dies aus fertigungstechnischer Sicht erforderlich oder vorteilhaft sein sollte.

Die oben beschriebenen Bauteile werden, gegebenenfalls nach dem Zusammenfügen zu einem einheitlichen Bauteil, im konfektionierten Artikulator an den üblicherweise dafür vorgesehenen Befestigungsstellen angebracht. Auf diesen Bauteilen werden anschließend die Ober- und Unterkiefermodelle befestigt. Die Ober- und Unterkiefermodelle sind damit zur Scharnierachse des Artikulators therapeutisch ausgerichtet.

Ein weiterer Gegenstand der Erfindung betrifft ein Verfahren zur patientenindividuellen Modifikation eines konfektionierten Artikulators umfassend die folgenden Schritte:
1) Berechnung eines patientenindividuellen Bauteils oder mehrerer patientenindividueller Bauteile (Bausatz) zur Modifikation eines konfektionierten Artikulators, unter Verwendung des zuvor definierten Verfahrens,
2) Erzeugung der in Schritt 1) berechneten patientenindividuellen Bauteile,
3) Einbau der in Schritt 1) erzeugten patientenindividuellen Bauteile in den konfektionierten Artikulator.

Zur Durchführung der Berechnung gemäß Schritt 1), wird zunächst der Abstand und die Ausrichtung der Scharnierachse des Artikulators zu den üblicherweise am Artikulator vorgesehenen Befestigungsstellen für Aufsätze zur Einpassung/Fixierung (Artikulierung) der Gebissregistrate ermittelt. Diese können beispielsweise durch manuelles Ausmessen der hierfür benötigten Distanzen und Winkel am betreffenden Artikulator erhalten werden. Diese können aber auch, falls vorhanden, aus digitalen Datensätzen, wie beispielsweise aus einem CAD-Datensatz des betreffenden konfektionierten Artikulators, herausgelesen werden. Hierzu kann auf allgemein erhältliche Software zurückgegriffen werden, die sich zur Darstellung dreidimensionaler digitaler Daten eignet.

Anhand der ermittelten Informationen zum Artikulator wird dann, wie bereits oben beschrieben, wenigstens ein patientenindividuelles Bauteil zur Modifikation des konfektionierten Artikulators berechnet, unter Verwendung des zuvor beschriebenen Verfahrens zur Bestimmung der therapeutischen Lage und Ausrichtung wenigstens eines anatomischen Symmetrieelements des Patienten.

Abhängig von der gewünschten Fertigungsart und der Konstruktion des konfektionierten Artikulators, bestehen die berechneten patientenindividuellen Bauteile, wie bereits oben beschrieben, aus einem einheitlichen Formkörper oder aus zwei oder mehr als zwei Teilen.

Im nachfolgenden Schritt 2) werden die berechneten Bauteile erzeugt. Hierzu eignen sich üblicherweise alle gängigen Verfahren zur Herstellung formstabiler Formkörper.

In Schritt 3) werden die in Schritt 2) erzeugten Bauteile an dem konfektionierten Artikulator, üblicherweise an den hierfür vorgesehenen Befestigungsstellen, befestigt. Die Befestigung erfolgt entweder, falls vorhanden, an den dafür vorgesehenen Halterungen, oder unter Zuhilfenahme geeigneter Befestigungsmittel, wie beispielsweise Kleber, Gips oder Zement.

Die nachfolgenden Figuren dienen der weiteren Erläuterung der Erfindung.
Figuren 1 und 2:

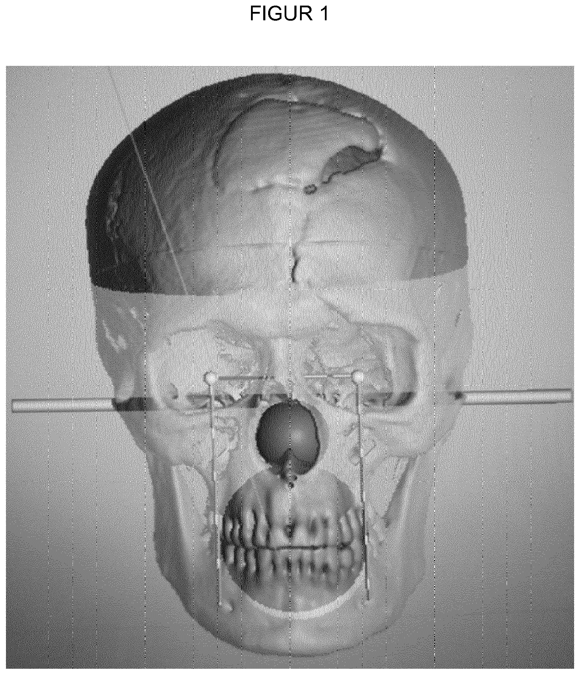

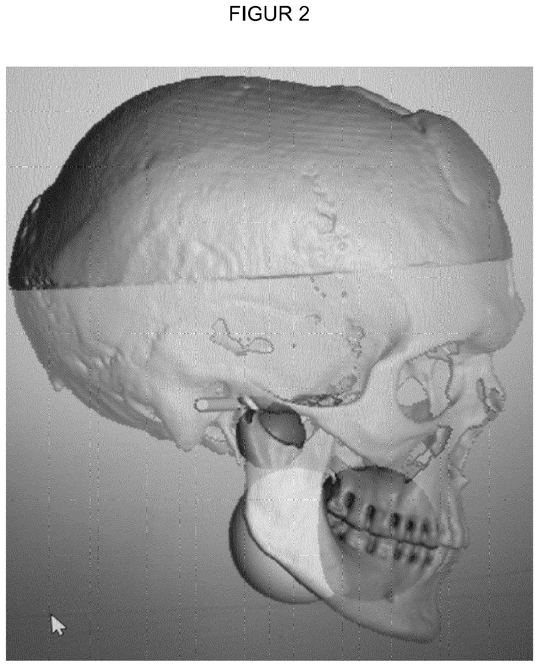

   Die Figuren 1 und 2 zeigen exemplarisch in Front- und Seitenansicht die mit Hilfe des erfindungsgemäßen Verfahrens anhand der Mittelpunkte der Pupillen (A) eines Patienten berechneten anatomischen Symmetrieelemente: Gaumenkugel (B), Rachenkugel (C), Kondylenkugel (D) und Kiefergelenksachse (E). Zur besseren Illustration wurden die berechneten anatomischen Symmetrieelemente mit der dreidimensionalen Knochenstruktur des Schädels des Patienten überlagert.
Figuren 3 und 4:

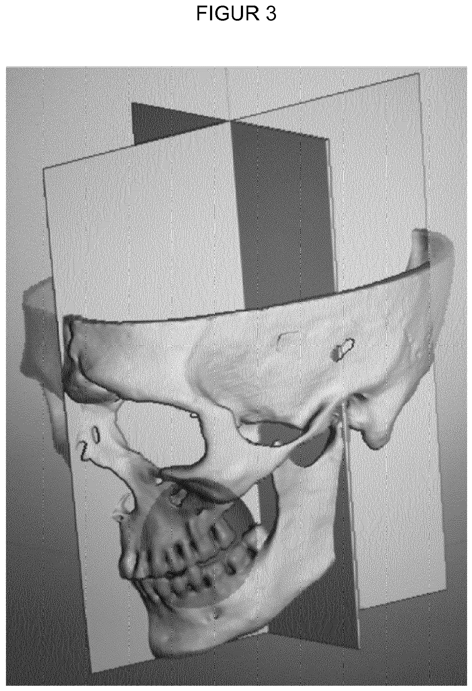

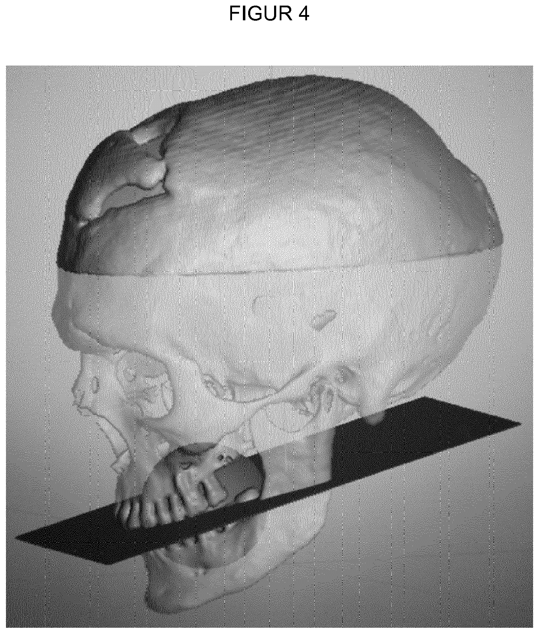

   Die Figuren 3 und 4 zeigen exemplarisch weitere durch das erfindungsgemäße Verfahren erhältliche anatomischen Symmetrieelemente: Gaumenkugel (B), craniale Symmetrieebene (F), Mittelmeridian (G) und Okklusionsebene (H). Auch hier wurden die berechneten anatomischen Symmetrieelemente zur besseren Illustration mit der dreidimensionalen Knochenstruktur des Schädels des Patienten überlagert.
Figur 5:

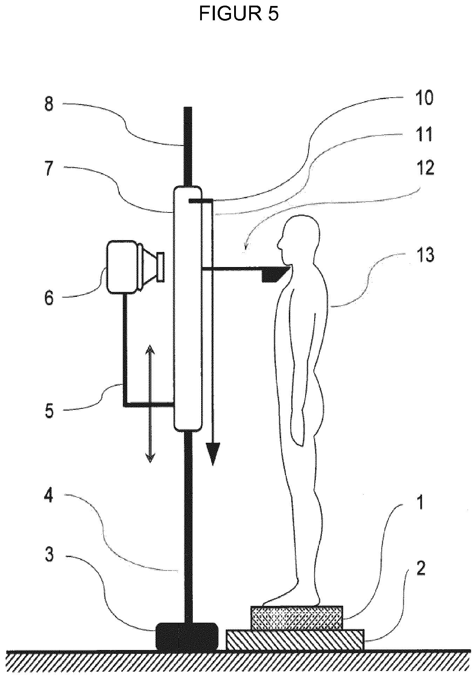

   Die Figuren 5 zeigt eine Ausführungsform der erfindungsgemäßen Vorrichtung zur Sicherstellung einer ausbalancierten Körperstatik des Patienten in aufrechter gerader Position und zur Erstellung eines biometrischen Fotos der Vorderansicht des Kopfes eines Patienten, enthaltend eine viskoelastische Standunterlage bzw. "Balance-Pad" (1), eine waagerecht ausrichtbare Bodenplatte (2), ein höhenverstellbares Stativ (4), welches auf einer Basisplatte (3) montiert ist, eine digitale Industriekamera (6), welche über eine Halterung (5) an dem höhenverstellbaren Stativ (4) angebracht ist, ein über eine Aufhängung (8) an dem höhenverstellbaren Stativ (4) angebrachter Einwegspiegel (7), einem Lot (11), welches ebenfalls über eine Halterung (10) an dem höhenverstellbaren Stativ (4) angebracht ist und einer höhenverstellbare Orientierungshilfe für die waagerechte Ausrichtung des Unterkiefers des Patienten (12).
   Die viskoelastische Standunterlage (1), auf die sich der Patient (13) in aufrechter gerader Position stellt, liegt auf der waagerecht ausrichtbaren Bodenplatte (2). Die Basisplatte (3) mit dem Stativ (4) ist daneben angeordnet.
   Das Stativ (4) ist so ausgestaltet, dass zwischen der digitalen Industriekamera (6) und dem Kopf des Patienten (13) eine freie Sicht durch den Einwegspiegel (7) gewährleistet ist.
Figur 6:

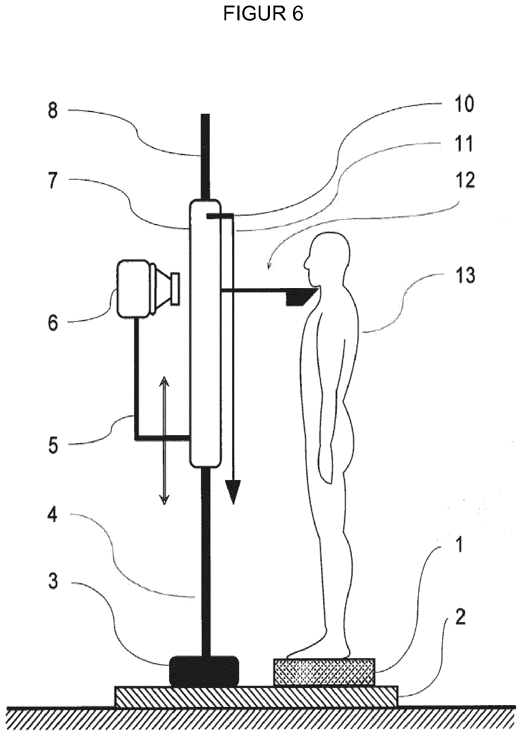

   Die Figuren 6 zeigt eine weitere Ausführungsform der erfindungsgemäßen Vorrichtung, welches dieselben zuvor unter Figur 5 aufgeführten Elemente (1) bis (12) aufweist, mit dem Unterschied, dass neben der viskoelastischen Standunterlage (1) auch die Basisplatte (3) mit dem Stativ (4) auf der waagerecht ausrichtbaren Bodenplatte (2) positioniert ist. Damit wird sichergestellt, dass nicht nur der Patient (13), sondern auch die Vorrichtung selbst auf einem waagerechten Untergrund steht.
Figuren 7A,

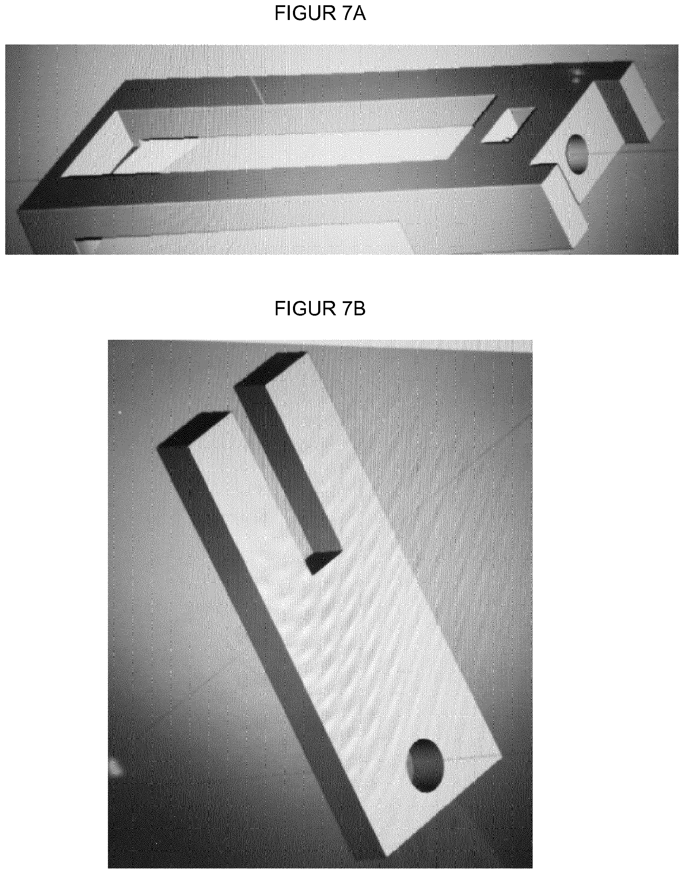

   Figuren 7A bis 7D zeigen exemplarisch vier Bauteile eines erfindungsgemäßen Bausatzes für die patientenindividuelle Modifikation bzw. des patientenindividuellen Umbaus des handelsüblichen Artikulators der Firma Baumann Dental (Artex Carbon Baureihe).
Figur 7A zeigt ein erstes Bauteil, das nicht patientenindividuell angefertigt wird und auf den Ebenenhalter des Artikulators montiert wird. Dieses Bauteil bleibt immer gleich, d. h. es wird nur einmal hergestellt.
Figur 7B zeigt ein zweites Bauteil, das nicht patientenindividuell angefertigt wird und als dorsaler Anschlag für die daran befestigten patientenindividuellen Bauteile dient. Auch dieses Bauteil bleibt immer gleich, d. h. es wird nur einmal hergestellt.

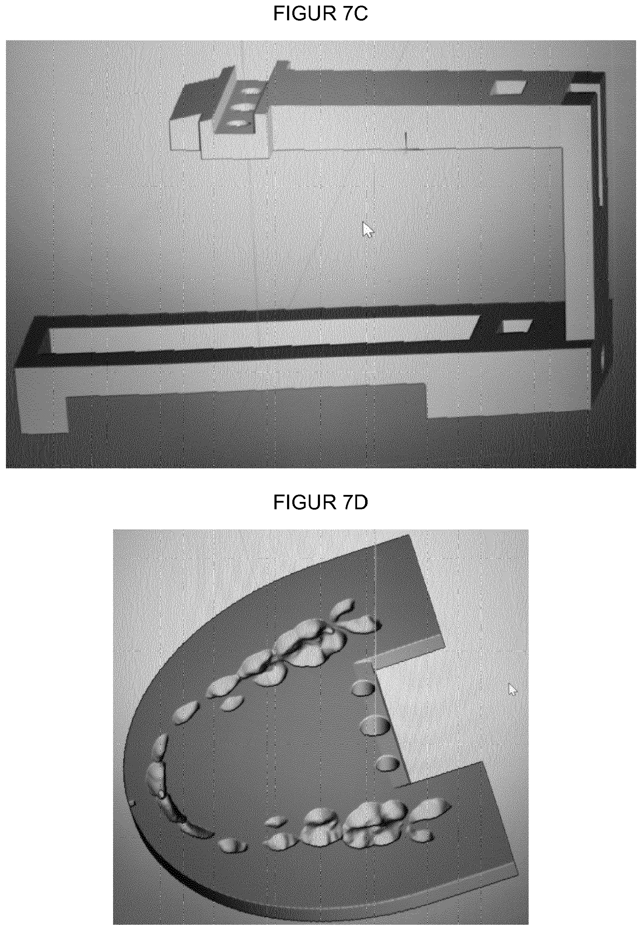

Figur 7C zeigt ein erstes patientenindividuelles Bauteil, das an die in den Figuren 7A und 7B gezeigten nicht-patientenindividuellen Bauteile angebracht ist. Dieses Bauteil legt den therapeutischen Abstand der Kiefermodellpaare zur Rotationsachse des Artikulators fest. Dieses Bauteil wird für jeden Patienten neu berechnet und gefertigt.
Figur 7D zeigt ein zweites patientenindividuelles Bauteil (Artikulationsplatte, die der therapeutischen Kauebene des Patienten entspricht) mit Impressionen der Ober- und Unterkiefermodelle. Diese Artikulationsplatte wird an das erste patientenindividuelle Bauteil befestigt und legt die räumliche Ausrichtung der Kiefermodelle (Rotation entlang der X-, Y- und Z-Achse) relativ zur Scharnierachse des Artikulators fest. Auch dieses Bauteil wird für jeden Patienten neu berechnet und gefertigt.

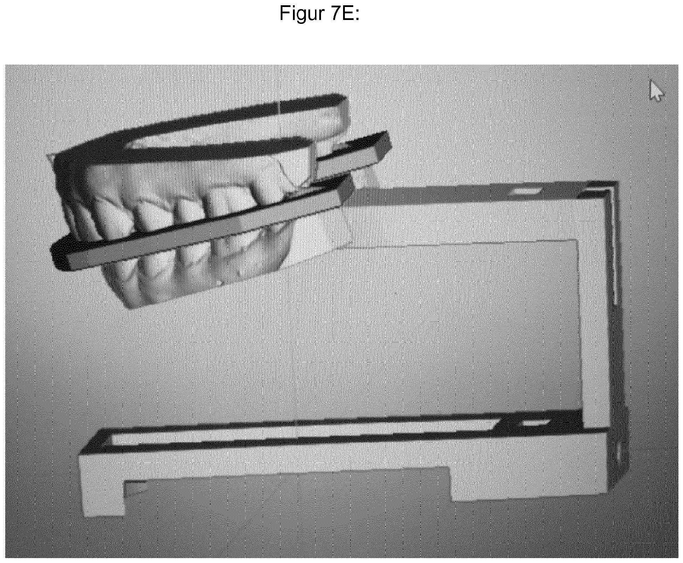

Figur 7E zeigt alle vier Bauteile zusammengefügt sowie die dazugehörigen therapeutisch ausgerichteten Ober- und Unterkiefermodelle.
Figur 8A, 8B und 8C:

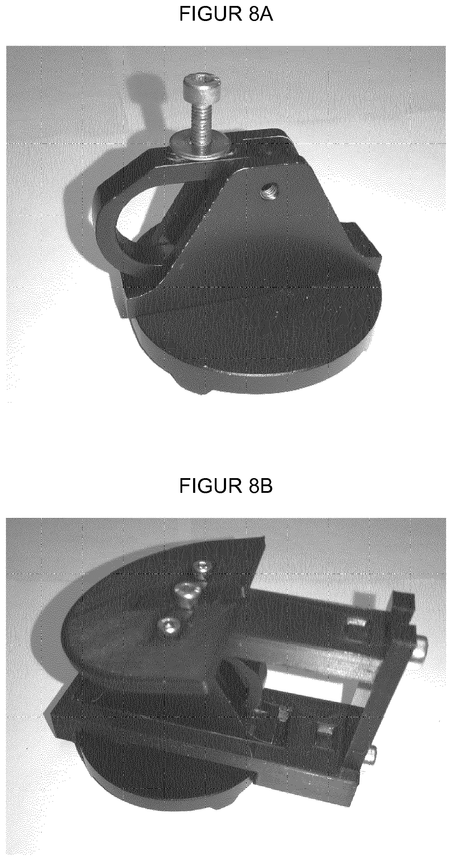

   Figur 8A zeigt einen Kalottenträger mit Sockelplatte für den handelsüblichen Artikulator Artex Carbon der Firma Baumann Dental auf dem der Bausatz für die patientenindividuelle Modifikation fixiert wird.
   Figur 8B zeigt den finalen gedruckten erfindungsgemäßen Bausatz zur patientenindividuellen Modifikation des handelsüblichen Artikulators der Firma Baumann Dental (Artex Carbon Baureihe), der auf dem in Figur 8A gezeigten Kalottenträger mit Sockelplatte montiert ist. Der Bausatz besteht aus den vier zusammengefügten Teilen (Bauteile 1, 2, 3 und 4), wie in den Abbildungen 7A bis 7E dargestellt, von denen die unteren beiden Bauteile (Bauteile 1 und 2) nur einmal gefertigt werden müssen und die daran befestigten oberen zwei Bauteile (Bauteile 3 und 4) patientenindividuell angefertigt werden. Bauteil 4 ist hier ohne Impressionen dargestellt.
      Die Form der beiden patientenindividuell gefertigten Bauteile 3 und 4 wurde durch das zuvor beschriebene erfindungsgemäße Verfahren ermittelt.

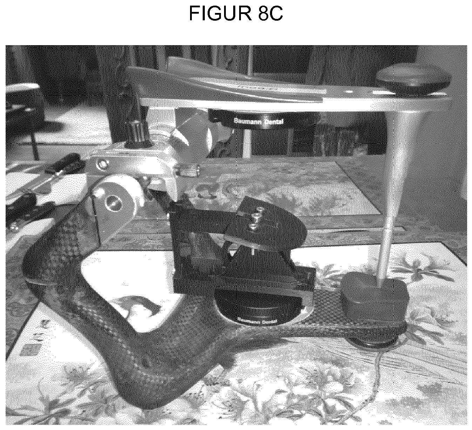

   Figur 8C zeigt einen Artikulator der Firma Baumann Dental (Artex Carbon Baureihe), der mit dem in Figur 8B gezeigten, auf dem Kalottenträger mit Sockelplatte montierten Bausatz modifiziert wurde. Bauteil 4 ist ohne Impressionen dargestellt.

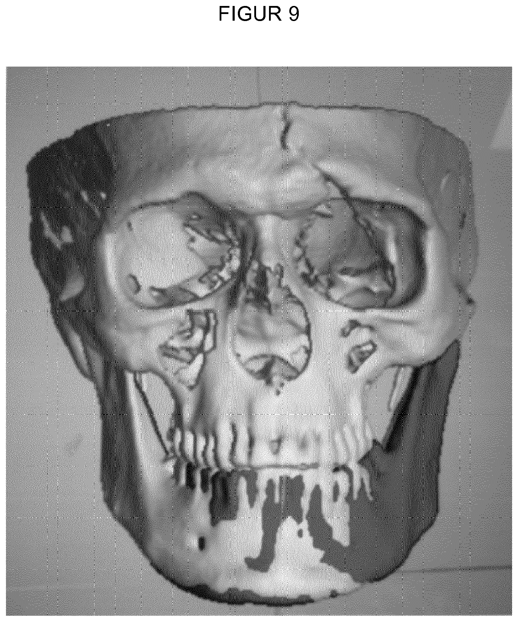

Figur 9 zeigt exemplarisch eine Überlagerung der Position des therapeutisch ausgerichteten Unterkiefermodells eines Patienten (dunkel gefärbt) mit der aktuellen Lage (Ist-Position) des Unterkiefers (hell gefärbt). Aus der Abbildung ist ersichtlich, dass bei der Behandlung eines Patienten mit wenigstens einer Erkrankung, die von einer funktionellen Störung des Kauorgans ausgeht, vergleichsweise kleine Änderungen der Lage der Kiefer zueinander entscheidend sind. Für das Gelingen solcher Therapie ist es daher absolut erforderlich, dass die therapeutische Lage der Kiefer zueinander mit sehr hoher Präzision bestimmt werden kann.

## Patentansprüche

1. Verfahren zur Bestimmung der therapeutischen Lage und Ausrichtung wenigstens eines anatomischen Symmetrieelements eines Patienten, umfassend die folgenden Schritte:
a) Sicherstellung einer ausbalancierten Körperstatik des Patienten in aufrechter gerader Position,
b) Erstellen eines biometrischen Fotos der Frontansicht des Kopfes des Patienten, und
c) computergestützte Berechnung der therapeutischen Lage und Ausrichtung wenigstens eines anatomischen Symmetrieelements des Patienten anhand des in Schritt b) erstellten biometrischen Fotos unter Zuhilfenahme einer mathematischen Matrix.

2. Verfahren nach Anspruch 1, wobei die Sicherstellung einer ausbalancierten Körperstatik des Patienten in Schritt a) wenigstens eine der folgenden Maßnahmen umfasst:
a.1) Einsatz einer Standunterlage zum Ausgleich der Längendifferenz der Beine,
a.2) Ausrichtung der Kopf- und/oder Körperhaltung unter Zuhilfenahme eines Lots,
a.3) gegebenenfalls die waagerechte Ausrichtung des Unterkiefers, und
a.4) Einsatz einer flexiblen Kaumaße zur Entspannung der Kaumuskulatur und zur Sicherstellung einer entspannten Unterkieferposition (Ruheschwebelage).

3. Verfahren nach einem der vorhergehenden Ansprüche, wobei das Verfahren zusätzlich wenigstens eines der folgenden Merkmale I) bis III) aufweist:
I) in Schritt a) werden zusätzlich Daten zur Körperstatik und zur Beweglichkeit des Patienten erfasst;
II) das in Schritt b) erstellte biometrische Foto ist ein zweidimensionales Foto;
III) das wenigstens eine anatomische Symmetrieelement ist ausgewählt unter Kiefergelenkachse, Halswirbelachsen (Atlasachsen), Körpermittelachse (Mittelmeridian), Gaumenkugel, Rachenkugel, Kondylenkugel, Atlaskugel, Kau-ebene, Atlasebene, kraniale Symmetrieebene, Frontalebene, Saggitalebene und Transversalebene.

4. Verfahren nach Anspruch 3, wobei die in Schritt a) erfassten Daten zur Körperstatik und zur Beweglichkeit des Patienten ausgewählt sind unter:
- Analyse der Ausrichtung und des Verlaufs der Wirbelsäule,
- Analyse der Beweglichkeit des Kopfes.

5. Verfahren nach einem der vorhergehenden Ansprüche, wobei der Schritt c) die folgenden computergestützten Schritte umfasst:
c.1) Nachbearbeitung des in Schritt b) erstellten biometrischen Fotos,
c.2) Bestimmung der für die Berechnung der therapeutischen Lage und Ausrichtung wenigstens eines anatomischen Symmetrieelements des Patienten nötigen biometrischen Abstände und Winkel aus dem in Schritt c.1) nachbearbeiteten biometrischen Foto,
c.3) Übertragung der in Schritt c.2) bestimmten biometrischen Abstände und Winkel in eine mathematische Matrix, und
c.4) Berechnung der therapeutischen Lage und Ausrichtung wenigstens eines anatomischen Symmetrieelements des Patienten.

6. Verwendung des in den Ansprüchen 1 bis 5 definierten Verfahrens für die Herstellung patientenindividueller Aufbissschienen, für die Planung und Berechnung von Zahnprothetik und für die patientenindividuelle Modifikation von Artikulatoren.

7. Verfahren zur Herstellung von Aufbissschienen zur Verwendung in der Behandlung eines Patienten mit wenigstens einer Erkrankung, die von einer funktionellen Störung des Kauorgans ausgeht, umfassend die folgenden Schritte:
i) computergestützte Bestimmung der therapeutischen Lage und Ausrichtung wenigstens eines für die Stellung der Kiefer relevanten anatomischen Symmetrieelements des Patienten nach einem der Ansprüche 1 bis 7,
ii) computergestützte lagerichtige Zuordnung digitalisierter Ober- und Unterkiefermodelle zu den in Schritt i) bestimmten Symmetrieelementen, und
iii) Erzeugung wenigstens eines STL-Datensatzes der Aufbissschiene.

8. Vorrichtung zur Sicherstellung einer ausbalancierten Körperstatik des Patienten in aufrechter gerader Position und zur Erstellung eines biometrischen Fotos der Vorderansicht des Kopfes eines Patienten, umfassend:
- eine erste digitale Kamera zur Aufnahme eines biometrischen Fotos der Frontansicht des Kopfes des Patienten in aufrechter gerader Position,
- ein Mittel zum Ausgleich der Längendifferenz der Beine des Patienten,
- wenigstens ein Mittel zum Auffinden einer ausbalancierten Körperstatik des Patienten, und
- wenigstens ein Mittel zur waagerechten Ausrichtung der Vorrichtung.

9. Vorrichtung nach Anspruch 8, wobei die Vorrichtung zusätzlich wenigstens eines der folgenden Merkmale A) bis F) aufweist:
A) die erste digitale Kamera und das wenigstens eine Mittel zum Auffinden der ausbalancierten Körperstatik des Patienten sind an einem Stativ angebracht;
B) die erste digitale Kamera ist ausgewählt unter Industriekameras;
C) als Mittel zum Ausgleich der Längendifferenz der Beine des Patienten wird eine viskoelastische Standunterlage eingesetzt;
D) als Mittel zum Auffinden der ausbalancierten Körperstatik des Patienten wird wenigstens ein Lot eingesetzt;
E) bei dem wenigstens einen Mittel zur waagerechten Ausrichtung der Vorrichtung handelt es sich um eine waagerecht ausrichtbare Bodenplatte.

10. Vorrichtung nach Anspruch 9, wobei als Mittel zum Auffinden der ausbalancierten Körperstatik des Patienten zusätzlich ein Einwegspiegel zwischen der ersten digitalen Kamera und dem Patienten positioniert ist.

11. Vorrichtung nach Anspruch 9, wobei das Lot vor dem Einwegspiegel und dem Patienten positioniert ist.

12. Vorrichtung nach einem der Ansprüche 8 bis 11, wobei die Vorrichtung zusätzlich eine oder mehrere der folgenden Mittel umfasst:
- Wirbelsäulen-Scanner zur Erfassung der Ausrichtung und des Verlaufs der Wirbelsäule des Patienten,
- eine zweite digitale Kamera zur Erfassung der Kopfbeweglichkeit des Patienten,
- eine Orientierungshilfe für die waagerechte Ausrichtung des Unterkiefers.

13. Vorrichtung nach einem der Ansprüche 8 bis 12 zur Verwendung in einem Verfahren nach einem der Ansprüche 1 bis 7.

14. Bausatz für die patientenindividuelle Modifikation eines konfektionierten Artikulators, umfassend:
- wenigstens ein Bauteil zur Fixierung des therapeutischen Abstandes der Kiefermodelle zur Scharnierachse des Artikulators,
- wenigstens ein Bauteil zur Fixierung der therapeutischen Ausrichtung der Kiefermodelle zur Scharnierachse des Artikulators.

15. Verfahren zur patientenindividuellen Modifikation eines konfektionierten Artikulators, umfassend die folgenden Schritte:
1) Berechnung eines patientenindividuellen Bauteils oder mehrerer patientenindividueller Bauteile (Bausatz) zur Modifikation eines konfektionierten Artikulators, unter Verwendung des in den Ansprüchen 1 bis 10 definierten Verfahrens,
2) Erzeugung der in Schritt 1) berechneten patientenindividuellen Bauteile,
3) Einbau der in Schritt 2) erzeugten patientenindividuellen Bauteile in den konfektionierten Artikulator.
